(19) Europäisches Patentamt
European Patent Office
Office européen des brevets

(11) EP 4 524 225 A2

(12) EUROPEAN PATENT APPLICATION

(43) Date of publication:
**19.03.2025 Bulletin 2025/12**

(21) Application number: **24219797.8**

(22) Date of filing: **26.02.2020**

(51) International Patent Classification (IPC):
***C11D 3/386*** *(2006.01)*

(52) Cooperative Patent Classification (CPC):
**C11D 3/386; C12N 9/54; C12Y 304/21062**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2019 EP 19160301**

(62) Document number(s) of the earlier application(s) in accordance with Art. 76 EPC:
**20705768.8 / 3 931 315**

(71) Applicant: **Novozymes A/S**
**2880 Bagsvaerd (DK)**

(72) Inventors:
- **KNÖTZEL, Jürgen, Carsten, Franz**
**2880 Bagsvaerd (DK)**
- **LUE, Bena-Marie**
**2880 Bagsvaerd (DK)**
- **JENSEN, Kenneth**
**2880 Bagsvaerd (DK)**

(74) Representative: **NZ EPO Representatives**
**Krogshoejvej 36**
**2880 Bagsvaerd (DK)**

Remarks:
•The complete document including Reference Table(s) and the Sequence Listing(s) can be downloaded from the EPO website
•This application was filed on 13.12.2024 as a divisional application to the application mentioned under INID code 62.

# (54) PROTEASE VARIANTS AND DETERGENT COMPOSITIONS COMPRISING SAME

(57) The invention relates to protease variants, detergent compositions comprising said variants, and use of said variants in cleaning processes.

## Description

### REFERENCE TO A SEQUENCE LISTING

This application contains a sequence listing in computer readable form, which is incorporated herein by reference.

### FIELD OF THE INVENTION

The present invention relates to protease variants, detergent compositions comprising said variants and to use of said variants in cleaning processes.

### BACKGROUND OF THE INVENTION

Subtilisins are serine proteases from the family S8, in particular from the subfamily S8A, as defined by the MEROPS database (https://www.ebi.ac.uk/merops/index.shtml). In subfamily S8A the key active site residues Asp, His and Ser are typically found in motifs that differ from those of the S8B subfamily.

In the detergent industry, enzymes have for many decades been implemented in detergent compositions for use in laundry or in hard surface cleaning such as dishwashing. Enzymes used in such compositions comprise proteases, lipases, amylases, cellulases, mannosidases as well as other enzymes or mixtures thereof. Commercially, the most important enzymes are proteases.

It is known that different protease enzymes may perform differently on different types of stains. There have been attempts to address this and improve wash performance by including two different proteases in a single detergent composition. Such compositions are disclosed in e.g. WO 2009/021867, WO 2014/177430, WO 2016/000970 and WO 2016/000971.

However, while some such compositions comprising two different proteases are disclosed in the patent literature, in practice, detergent compositions comprising two different proteases have not been met with any widespread commercial success.

Further, the relatively few compositions comprising two proteases that are described in the patent literature are disclosed in the context of automatic dishwashing. In contrast, there has not been a focus on developing detergent compositions with two different proteases adapted to the conditions and requirements found in laundry.

Thus, given that individual protease enzymes used in currently available detergent compositions are not able to effectively remove all relevant protein-based stains in any given setting, especially in laundry settings, there remains a need for detergent compositions with improved protein stain-removal properties.

The present invention addresses this need by providing detergent compositions comprising at least two different protease enzymes with different net formal charge characteristics, where the combination of the two proteases results in an improved wash performance on individual stains and/or covers a broader set of stains compared to the individual enzymes. It is contemplated that the compositions of the invention will be particularly useful as laundry detergent compositions, and especially in liquid laundry detergent compositions.

### SUMMARY OF THE INVENTION

The present invention relates to novel protease variants with advantageous properties, e.g. improved wash performance, when such proteases are used either alone as a single protease or in detergent compositions with another protease, where the proteases are variants of SEQ ID NO: 1 comprising substitutions in positions 97, 209, and 215.

In a first aspect, the present invention relates to protease variants comprising the substitutions X97D, X209W and X215K (e.g. G97D, Y209W and A215K), wherein position numbers are based on the numbering of SEQ ID NO: 2, and wherein the variants have protease activity and have at least 80% but less than 100% sequence identity to SEQ ID NO: 1.

In a second aspect, the present invention relates to detergent compositions comprising variants of the first aspect.

In a third aspect, the present invention relates to use of a protease variant according to the first aspect in a cleaning process, preferably laundry.

### OVERVIEW OF SEQUENCES

SEQ ID NO: 1 is the sequence of the Savinase® protease polypeptide from *Bacillus lentus.*

SEQ ID NO: 2 is the sequence of the BPN' protease polypeptide from *Bacillus amyloliquefaciens.*

## BRIEF DESCRIPTION OF FIGURES

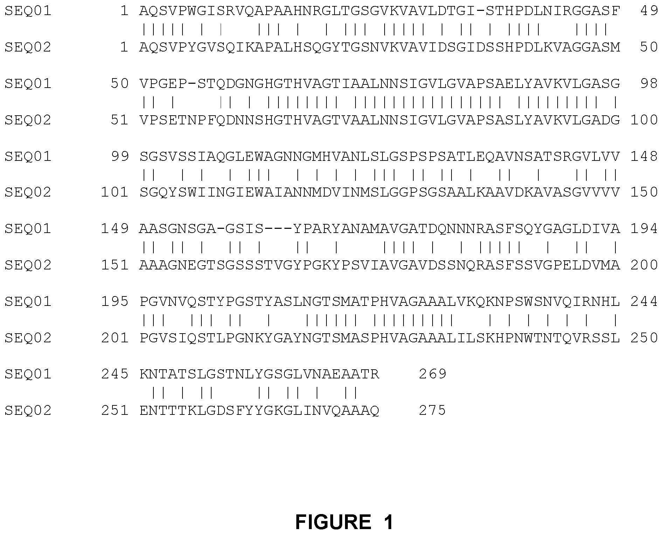

Figure 1 is an alignment of the amino acid sequences of subtilisin 309 (SEQ ID NO: 1) and subtilisin BPN' (SEQ ID NO: 2).

## DEFINITIONS

**Subtilase/protease:** The terms "subtilase" and "protease" may be used interchangeably herein and refer to an enzyme that hydrolyses peptide bonds in proteins. This includes any enzyme belonging to the EC 3.4 enzyme group (including each of the thirteen subclasses thereof), and in particular endopeptidases (EC 3.4.21). The EC number refers to Enzyme Nomenclature 1992 from NC-IUBMB, Academic Press, San Diego, California, including supplements 1-5 published in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650; respectively.

**Protease activity:** The term "protease activity" means a proteolytic activity (EC 3.4), in particular endopeptidase activity (EC 3.4.21). There are several protease activity types, the three main activity types being: trypsin-like, where there is cleavage of amide substrates following Arg or Lys at P1, chymotrypsin-like, where cleavage occurs following one of the hydrophobic amino acids at P1, and elastase-like with cleavage following an Ala at P1. Protease activity may be determined according to the procedure described in WO 2016/087619.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment - Total Number of Gaps in Alignment)

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice *et al.,* 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment - Total Number of Gaps in Alignment)

**Variant:** The term "variant" means a polypeptide having protease activity comprising an alteration, *i.e.,* a substitution, insertion, and/or deletion, at one or more positions. A substitution means replacement of the amino acid occupying a position with a different amino acid; a deletion means removal of the amino acid occupying a position; and an insertion means adding an amino acid adjacent to and immediately following the amino acid occupying a position.

### Conventions for Designation of Variants

For purposes of the present invention, the polypeptide of SEQ ID NO: 2 is used to determine the corresponding amino acid residue number in a variant of SEQ ID NO: 1. The amino acid sequence of a variant of SEQ ID NO: 1 is aligned with SEQ ID NO: 2, and based on the alignment, the amino acid position number corresponding to any amino acid residue in the polypeptide of SEQ ID NO: 1 is determined. See the paragraph "Numbering of amino acid positions/residues" below for further information.

Identification of the corresponding amino acid residue in another subtilase can be determined by an alignment of multiple polypeptide sequences using several computer programs including, but not limited to, MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic

Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680), using their respective default parameters.

In describing the variants of the present invention, the nomenclature described below is adapted for ease of reference. The accepted IUPAC single letter or three letter amino acid abbreviation is employed. The terms "alteration" or "mutation" may be used interchangeably herein to refer to substitutions, insertions and deletions.

Substitutions. For an amino acid substitution, the following nomenclature is used: Original amino acid, position, substituted amino acid. For example, the substitution of a threonine at position 220 with alanine is designated as "T220A". Multiple substitutions may be separated by addition marks ("+"), e.g., "T220A + G229V", representing substitutions at positions 220 and 229 of threonine (T) with alanine (A) and glycine (G) with valine (V), respectively. Multiple substitutions may alternatively be listed with individual mutations separated by a space or a comma. Alternative substitutions in a particular position may be indicated with a slash ("/"). For example, substitution of threonine in position 220 with either alanine, valine or leucine many be designated "T220A/V/L". An "X" preceding a position means that any original amino acid at the position may be substituted. For example, X9E means that any amino acid residue at position 9 other than E is substituted with E.

Deletions. For an amino acid deletion, the following nomenclature is used: Original amino acid, position, *. Accordingly, the deletion of threonine at position 220 is designated as "T220*". Multiple deletions may be separated by addition marks ("+"), e.g., "T220* + G229*", or alternatively may be separated by a space or comma. The use of an "X" preceding a position number is as described above for substitutions, e.g. "X131*" means that the amino acid residue at position 131 is deleted.

Insertions. For an amino acid insertion, the following nomenclature is used: Original amino acid, position, original amino acid, inserted amino acid. Accordingly, the insertion of lysine after threonine at position 220 is designated "T220TK". An insertion of multiple amino acids is designated [Original amino acid, position, original amino acid, inserted amino acid #1, inserted amino acid #2; etc.]. For example, the insertion of lysine and alanine after threonine at position 220 is indicated as "T220TKA".

In such cases the inserted amino acid residue(s) are numbered by the addition of lower case letters to the position number of the amino acid residue preceding the inserted amino acid residue(s). In the above example, the sequence would thus be:

| Parent: | Variant: |
|---|---|
| 220 | 220 220a 220b |
| T | T - K - A |

Multiple alterations. Variants comprising multiple alterations are separated by addition marks ("+"), e.g., "R170Y+G195E" representing a substitution of arginine and glycine at positions 170 and 195 with tyrosine and glutamic acid, respectively. Multiple alterations may alternatively be listed with individual mutations separated by a space or a comma.

A combination of e.g. a substitution and an insertion may be denoted as follows: S99AD, which represents substitution of a serine residue in position 99 with an alanine residue as well as insertion of an aspartic acid residue.

Different alterations. Where different alterations can be introduced at a position, the different alterations may be separated by a comma, e.g., "R170Y,E" represents a substitution of arginine at position 170 with tyrosine or glutamic acid. Thus, "Y167G,A + R170G,A" designates the following variants:
"Y167G+R170G", "Y167G+R170A", "Y167A+R170G", and "Y167A+R170A".

Different alterations in a position may also be indicated with a slash ("/"), for example "T220A/V/L" as explained above. Alternatively, different alterations may be indicated using brackets, e.g., R170 [Y,G].

Numbering of amino acid positions/residues. The numbering used herein is based on the numbering of SEQ ID NO: 2. Thus, amino acid residues of SEQ ID NO: 1 are numbered based on the corresponding amino acid residue in SEQ ID NO: 2. Specifically, the numbering is based on the alignment in Table 1 of WO 89/06279, which shows an alignment of five proteases, including the mature polypeptide of the subtilase BPN' (BASBPN) sequence (sequence c in the table) and the mature polypeptide of subtilisin 309 from *Bacillus lentus,* also known as Savinase® (BLSAVI) (sequence a in the table). Persons skilled in the art will know that position numbers used for subtilisin 309 and other proteases in the patent literature are often based on the corresponding position numbers of BPN' according to this alignment.

The accompanying Figure 1 is provided for reference purposes and shows an alignment between SEQ ID NO: 1 and SEQ ID NO: 2, based on Table 1 of WO 89/06279, from which position numbers corresponding to positions of SEQ ID NO: 2 may be readily determined.

## DETAILED DESCRIPTION OF THE INVENTION

The present invention relates to detergent compositions comprising at least a first protease and a second protease, where the first protease and the second protease have different net formal charges relative to the protease of SEQ ID NO: 1.

The term "net formal charge", or "net charge", refers to the net charge of the enzyme, where the amino acid residues aspartic acid (D) and glutamic acid (E) contribute to one negative charge (-1), and the amino acid residues arginine (R) and lysine (K) contribute to one positive charge (+1), and where the charge of these residues in the first protease or second protease is compared to the charge of the residues in the protease of SEQ ID NO: 1. This means that replacing a neutral amino acid, e.g. a neutral amino acid in SEQ ID NO: 1, with an acidic amino acid (D or E) provides one negative charge, while replacing a neutral amino acid with a basic amino acid (R or K) provides one positive charge. Similarly, replacing an acidic amino acid with a basic amino acid would provide two positive charges, while replacing a basic amino acid with an acidic amino acid would provide two negative charges.

As explained above, the net charge of a protease in the detergent compositions of the invention is expressed relative to the net charge of the protease of SEQ ID NO: 1. It may be seen from the sequence of SEQ ID NO: 1 that this protease contains 5 Asp residues, 5 Glu residues, 8 Arg residues and 5 Lys residues, thus SEQ ID NO: 1 has a net charge of +3, and it is this value to which the first and second proteases of the invention are to be compared. The net charge of a protease in the compositions of the invention may thus be readily determined by counting the number of acidic and basic amino acids in the amino acid sequence and comparing the calculated net charge with the net charge of the protease of SEQ ID NO: 1. It will be apparent that the net charge of the first and the second protease determined in this manner is a function of the number of acidic (D and E) and basic (R and K) amino acid residues and is for purposes of the invention independent of the composition having any particular pH value.

The invention relates in particular to a detergent composition comprising a first protease and a second protease, where

1) the first protease has a net formal charge of -1, -3 or -4 relative to the protease of SEQ ID NO: 1, and the second protease has a net formal charge of -2 relative to the protease of SEQ ID NO: 1; or
2) the first protease has a net formal charge of -3, -4 or -5 relative to the protease of SEQ ID NO: 1, and the second protease has a net formal charge of -1, 0 or +1 relative to the protease of SEQ ID NO: 1.

It will be apparent that the terms "first protease" and "second protease" are used herein to differentiate between the two proteases having different net charges in a given composition, and that a protease with a given net formal charge may in some cases, according to the context of the particular composition in which it is included, be considered as either a "first protease" or a "second protease". For example, a protease with a net formal charge of -1 may be referred to as a "first protease" when it is present in a composition comprising another protease with a net formal charge of -2, but may be a "second protease" when it is present in a composition comprising another protease with a net formal charge of -3, -4 or -5.

In one aspect, the invention thus relates to a detergent composition comprising a first protease and a second protease, where the first protease has a net formal charge of -1, -3 or -4 relative to the protease of SEQ ID NO: 1, and the second protease has a net formal charge of -2 relative to the protease of SEQ ID NO: 1. In a preferred embodiment of this aspect, the first protease has a net formal charge of -3 or -4.

In one embodiment, the first protease has a net formal charge of -1 relative to the protease of SEQ ID NO: 1, and the second protease has a net formal charge of -2 relative to the protease of SEQ ID NO: 1.

In one embodiment, the first protease has a net formal charge of -3 relative to the protease of SEQ ID NO: 1, and the second protease has a net formal charge of -2 relative to the protease of SEQ ID NO: 1.

In one embodiment, the first protease has a net formal charge of -4 relative to the protease of SEQ ID NO: 1, and the second protease has a net formal charge of -2 relative to the protease of SEQ ID NO: 1.

In another aspect, the invention relates to a detergent composition comprising a first protease and a second protease, where the first protease has a net formal charge of -3, -4 or -5 relative to the protease of SEQ ID NO: 1, and the second protease has a net formal charge of-1, 0 or +1 relative to the protease of SEQ ID NO: 1.

In one embodiment, the first protease has a net formal charge of -3 relative to the protease of SEQ ID NO: 1, and the second protease has a net formal charge of +1 relative to the protease of SEQ ID NO: 1.

In one embodiment, the first protease has a net formal charge of -4 relative to the protease of SEQ ID NO: 1, and the second protease has a net formal charge of +1 relative to the protease of SEQ ID NO: 1.

In one embodiment, the first protease has a net formal charge of -5 relative to the protease of SEQ ID NO: 1, and the second protease has a net formal charge of +1 relative to the protease of SEQ ID NO: 1.

In one embodiment, the first protease has a net formal charge of -3 relative to the protease of SEQ ID NO: 1, and the second protease has a net formal charge of 0 relative to the protease of SEQ ID NO: 1.

In one embodiment, the first protease has a net formal charge of -4 relative to the protease of SEQ ID NO: 1, and

the second protease has a net formal charge of 0 relative to the protease of SEQ ID NO: 1.

In one embodiment, the first protease has a net formal charge of -5 relative to the protease of SEQ ID NO: 1, and the second protease has a net formal charge of 0 relative to the protease of SEQ ID NO: 1.

In one embodiment, the first protease has a net formal charge of -3 relative to the protease of SEQ ID NO: 1, and the second protease has a net formal charge of -1 relative to the protease of SEQ ID NO: 1.

In one embodiment, the first protease has a net formal charge of -4 relative to the protease of SEQ ID NO: 1, and the second protease has a net formal charge of -1 relative to the protease of SEQ ID NO: 1.

In one embodiment, the first protease has a net formal charge of -5 relative to the protease of SEQ ID NO: 1, and the second protease has a net formal charge of -1 relative to the protease of SEQ ID NO: 1.

It will be apparent that the net formal charge difference between the first protease and the second protease in the compositions of the invention may vary, so that the net formal charge of the two proteases in a composition may differ by, e.g., 1, 2, 3, 4, 5, 6 or 7.

In certain preferred embodiments, the detergent composition of the invention comprises a first and a second protease that each have a negative net formal charge relative to the protease of SEQ ID NO: 1, for example a net formal charge of -1, -2, -3 or -4, and where the net formal charge of the first and second protease differ from each other by 1 or 2. Specific examples of such compositions include compositions comprising:

a) a first protease with a net formal charge of -4 and a second protease with a net formal charge of -2 relative to the protease of SEQ ID NO: 1 (charge difference of 2);
b) a first protease with a net formal charge of -3 and a second protease with a net formal charge of -2 relative to the protease of SEQ ID NO: 1 (charge difference of 1); and
c) a first protease with a net formal charge of -3 and a second protease with a net formal charge of -1 relative to the protease of SEQ ID NO: 1 (charge difference of 2).

Such compositions with two proteases having a net formal charge of -1, -2, -3 or -4 and a charge difference of 1 or 2 are contemplated to be particularly useful when formulated as a liquid laundry detergent composition.

As mentioned above, use of the combination of at least two different protease enzymes with different net formal charge characteristics has been found to result in an improved wash performance on individual stains and/or to provide improved overall performance on a broader set of stains, e.g. in laundry detergent formulations, compared to the individual enzymes. Use of this concept of different net formal charges of the individual proteases allows the blend to be tailored to different wash conditions to achieve an optimal charge blend for improved performance on targeted stains.

In particular, it has been found that by use of this concept it is possible, without increasing the total protease enzyme content, to provide increased cleaning performance on certain difficult to remove stains, for example oil-containing protein stains such as sebum stains, while at the same time substantially maintaining or improving performance on other protein-based stains, such as cocoa stains. The overall result is an improved cleaning performance on a broader stain set using the same amount of enzyme protein.

In another aspect, the invention relates to a detergent composition comprising a first protease and a second protease, where the first protease has a net formal charge of -3, -4 or -5 relative to the protease of SEQ ID NO: 1, and the second protease has a net formal charge of +2 relative to the protease of SEQ ID NO: 1.

In a further aspect, the invention relates to a detergent composition comprising a first protease and a second protease, where the first protease has a net formal charge of 0, -1 or -2 relative to the protease of SEQ ID NO: 1, and the second protease has a net formal charge of +2, +3, +4 or +5 relative to the protease of SEQ ID NO: 1. It is contemplated that such compositions wherein one protease has a relatively positive net formal charge relative to the protease of SEQ ID NO: 1 will provide advantageous results in relatively high pH wash settings, for example in automatic dishwashing applications.

In one embodiment, the first protease has a net formal charge of -3 relative to the protease of SEQ ID NO: 1, and the second protease has a net formal charge of +2 relative to the protease of SEQ ID NO: 1.

In one embodiment, the first protease has a net formal charge of -4 relative to the protease of SEQ ID NO: 1, and the second protease has a net formal charge of +2 relative to the protease of SEQ ID NO: 1.

In one embodiment, the first protease has a net formal charge of -5 relative to the protease of SEQ ID NO: 1, and the second protease has a net formal charge of +2 relative to the protease of SEQ ID NO: 1.

In one embodiment, the first protease has a net formal charge of 0 relative to the protease of SEQ ID NO: 1, and the second protease has a net formal charge of +2 relative to the protease of SEQ ID NO: 1.

In one embodiment, the first protease has a net formal charge of -1 relative to the protease of SEQ ID NO: 1, and the second protease has a net formal charge of +2 relative to the protease of SEQ ID NO: 1.

In one embodiment, the first protease has a net formal charge of -2 relative to the protease of SEQ ID NO: 1, and the second protease has a net formal charge of +2 relative to the protease of SEQ ID NO: 1.

In one embodiment, the first protease has a net formal charge of 0 relative to the protease of SEQ ID NO: 1, and the second protease has a net formal charge of +3 relative to the protease of SEQ ID NO: 1.

In one embodiment, the first protease has a net formal charge of -1 relative to the protease of SEQ ID NO: 1, and the second protease has a net formal charge of +3 relative to the protease of SEQ ID NO: 1.

In one embodiment, the first protease has a net formal charge of -2 relative to the protease of SEQ ID NO: 1, and the second protease has a net formal charge of +3 relative to the protease of SEQ ID NO: 1.

In one embodiment, the first protease has a net formal charge of 0 relative to the protease of SEQ ID NO: 1, and the second protease has a net formal charge of +4 relative to the protease of SEQ ID NO: 1.

In one embodiment, the first protease has a net formal charge of -1 relative to the protease of SEQ ID NO: 1, and the second protease has a net formal charge of +4 relative to the protease of SEQ ID NO: 1.

In one embodiment, the first protease has a net formal charge of -2 relative to the protease of SEQ ID NO: 1, and the second protease has a net formal charge of +4 relative to the protease of SEQ ID NO: 1.

In one embodiment, the first protease has a net formal charge of 0 relative to the protease of SEQ ID NO: 1, and the second protease has a net formal charge of +5 relative to the protease of SEQ ID NO: 1.

In one embodiment, the first protease has a net formal charge of -1 relative to the protease of SEQ ID NO: 1, and the second protease has a net formal charge of +5 relative to the protease of SEQ ID NO: 1.

In one embodiment, the first protease has a net formal charge of -2 relative to the protease of SEQ ID NO: 1, and the second protease has a net formal charge of +5 relative to the protease of SEQ ID NO: 1.

In any of the detergent compositions of the invention, the first protease and the second protease preferably both have an amino acid sequence that has at least 60% sequence identity to SEQ ID NO: 1, for example at least 70% or at least 80% sequence identity. The first protease may thus have at least 85% sequence identity to SEQ ID NO: 1, such as at least 90%, at least 91%, at least 92%, at least 93%, at least 94% or at least 95% sequence identity to SEQ ID NO: 1. Similarly, the second protease may have at least 85% sequence identity to SEQ ID NO: 1, such as at least 90%, at least 91%, at least 92%, at least 93%, at least 94% or at least 95% sequence identity to SEQ ID NO: 1. It will be understood that if such proteases comprise any additional mutations relative to SEQ ID NO: 1 beyond those specified herein, the proteases should fulfil the relevant net formal charge criteria in relation to SEQ ID NO: 1. The first and/or second protease may also have an amino acid sequence that comprises or consists of SEQ ID NO: 1 with the specific mutations defined herein.

In one embodiment, the first protease has a net formal charge of -5, -4 or -3 and comprises, relative to SEQ ID NO: 1, and comprises one or more mutations that introduce an increased negative charge selected from the group consisting of S9E, K27H, K27M, *36D, N62D, N76D, N77D, G97D, S99D, S101E, H120D, N140D, S156D, N185E, S188E, G195E, K235L, K235M, K237M, S259D and L262E. Preferred substitutions to introduce a negative charge include N76D, G97D, S99D, S101E and S156D. In preferred embodiments, the first protease comprises more than one of these mutations, preferably three or more, such as four, five or six of these mutations. In some embodiments, a variant having four or more of these mutations that provide an increased negative charge may be combined with one or more mutations, for example one or two mutations, that provide an increased positive charge, so as to result in a net formal charge relative to the protease of SEQ ID NO: 1 of -5, -4 or -3. Examples of such mutations to provide an increased positive charge include e.g. S9R, N43R, N117R, A215K and Q245R.

In one embodiment, the first protease thus comprises, relative to SEQ ID NO: 1, four or more mutations, such as four, five, six or seven mutations, that introduce an increased negative charge, combined with one or more mutations, such as one or two mutations, that introduce an increased positive charge, so as to result in a net formal charge of -5, -4 or -3 relative to the protease of SEQ ID NO: 1. The first protease may thus comprise four or more mutations that introduce an increased negative charge, e.g. selected from the group consisting of S9E, K27H, K27M, *36D, N62D, N76D, N77D, G97D, S99D, S101E, H120D, N140D, S156D, N185E, S188E, G195E, K235L, K235M, K237M, S259D and L262E, together with one or two mutations that introduce an increased positive charge, e.g. selected from the group consisting of S9R, N43R, N117R, A215K and Q245R, where the protease has a net formal charge of -5, -4 or -3 relative to the protease of SEQ ID NO: 1.

In all of the embodiments below and elsewhere herein comprising a protease which is a variant of SEQ ID NO: 1, position numbers are based on the numbering of SEQ ID NO: 2 in accordance with the explanation in the paragraph "Numbering of amino acid positions/residues" above.

In one embodiment, the first protease is a variant of SEQ ID NO: 1 comprising the substitutions S99D+S101E+S103A+V104I+S156D+G160S+L262E, i.e. having a net formal charge of -4 relative to SEQ ID NO: 1.

In one embodiment, the first protease is a variant of SEQ ID NO: 1 comprising the substitutions K27H+S99D+S101E+S103A+V104I+S156D+G160S+Q245R+L262E, i.e. having a net formal charge of -4 relative to SEQ ID NO: 1.

In one embodiment, the first protease is a variant of SEQ ID NO: 1 comprising the substitutions S9R+S99D+S101E+S103A+V104I+S156D+G160S+K235M+Q245R+L262E, i.e. having a net formal charge of -3 relative to SEQ ID NO: 1.

In one embodiment, the first protease is a variant of SEQ ID NO: 1 comprising the substitutions S9R+S99D+S101E+S103A+V104I+S156D+G160S+L262E, i.e. having a net formal charge of -3 relative to SEQ ID NO: 1.

In one embodiment, the first protease is a variant of SEQ ID NO: 1 comprising the substitutions S9R+K27M+S99D+S101E+S103A+V104I+S156D+G160S+K237M+Q245R+ L262E, i.e. having a net formal charge of -4 relative to SEQ ID NO: 1.

In one embodiment, the first protease is a variant of SEQ ID NO: 1 comprising the substitutions S9E+N43-R+N76D+V205I+Q206L+Y209W+S259D+N261W+L262E, i.e. having a net formal charge of -3 relative to SEQ ID NO: 1.

In one embodiment, the first protease is a variant of SEQ ID NO: 1 comprising the substitutions S9E+N43-R+N76D+N185E+S188E+Q191N+A194P+Q206L+Y209W+S259D+ L262E, i.e. having a net formal charge of -5 relative to SEQ ID NO: 1.

In one embodiment, the first protease is a variant of SEQ ID NO: 1 comprising the mutations *36D+N76D+H120D+G195E+K235L, i.e. having a net formal charge of -5 relative to SEQ ID NO: 1.

In one embodiment, the first protease is a variant of SEQ ID NO: 1 comprising the substitutions S9R+K27M+S99A+S101E+S103A+V104I+S156D+G160S+K237M+Q245R+ L262E, i.e. having a net formal charge of -3 relative to SEQ ID NO: 1.

In one embodiment, the first protease is a variant of SEQ ID NO: 1 comprising the substitutions S9R+K27M+S101E+S103A+V104I+S1560+G160S+K237M+Q245R+L262E, i.e. having a net formal charge of -3 relative to SEQ ID NO: 1.

In one embodiment, the first protease is a variant of SEQ ID NO: 1 comprising the substitutions S9R+K27M+S99D+S101E+S103A+V104I+S156D+G160S+A215K+K237M+ Q245R+L262E, i.e. having a net formal charge of -3 relative to SEQ ID NO: 1.

In one embodiment, the first protease is a variant of SEQ ID NO: 1 comprising the substitutions S9R+K27M+N43R+S99D+S101E+S103A+V104I+S156D+G160S+K237M+Q245R +L262E, i.e. having a net formal charge of -3 relative to SEQ ID NO: 1.

In one embodiment, the first protease is a variant of SEQ ID NO: 1 comprising the substitutions S9R+N43R+S99D+S101E+S103A+V104I+S156D+G160S+N173D+K235M+ Q245R+L262E, i.e. having a net formal charge of -3 relative to SEQ ID NO: 1.

In one embodiment, the first protease is a variant of SEQ ID NO: 1 comprising the substitutions S9E+G97D+S156D+Y209W+A215K+L262E, i.e. having a net formal charge of -3 relative to SEQ ID NO: 1.

In one embodiment, the first protease is a variant of SEQ ID NO: 1 comprising the substitutions G97D+S101E+S156D+A172V+Y209W+A215K+L262E, i.e. having a net formal charge of -3 relative to SEQ ID NO: 1.

In one embodiment, the first protease is a variant of SEQ ID NO: 1 comprising the substitutions N76D+G97D+N140D+S156D+Y209W+A215K+L262E, i.e. having a net formal charge of -4 relative to SEQ ID NO: 1.

In one embodiment, the first protease is a variant of SEQ ID NO: 1 comprising the substitutions N76D+G97D+S101E+T180A+Y209W+A215K+L262E, i.e. having a net formal charge of -3 relative to SEQ ID NO: 1.

In one embodiment, the first protease is a variant of SEQ ID NO: 1 comprising the substitutions N76D+G97D+S101E+Y209W+A215K+L262E, i.e. having a net formal charge of -3 relative to SEQ ID NO: 1.

In one embodiment, the first protease is a variant of SEQ ID NO: 1 comprising the substitutions N62D+G97D+S101E+Y209W+A215K+L262E, i.e. having a net formal charge of -3 relative to SEQ ID NO: 1.

In one embodiment, the first protease is a variant of SEQ ID NO: 1 comprising the substitutions N62D+G97D+S101E+V177I+Y209W+A215K+L262E, i.e. having a net formal charge of -3 relative to SEQ ID NO: 1.

In one embodiment, the first protease is a variant of SEQ ID NO: 1 comprising the substitutions N62D+N76D+G97D+Y209W+A215K+L262E, i.e. having a net formal charge of -3 relative to SEQ ID NO: 1.

In one embodiment, the first protease is a variant of SEQ ID NO: 1 comprising the substitutions K27M+G97D+S156D+Y209W+A215K+L262E, i.e. having a net formal charge of -3 relative to SEQ ID NO: 1.

In one embodiment, the first protease is a variant of SEQ ID NO: 1 comprising the substitutions K27M+N77D+G97D+S156D+Y209W+A215K+L262E, i.e. having a net formal charge of -4 relative to SEQ ID NO: 1.

In one embodiment, the first protease is a variant of SEQ ID NO: 1 comprising the substitutions K27M+N76D+G97D+Y209W+A215K+L262E, i.e. having a net formal charge of -3 relative to SEQ ID NO: 1.

In one embodiment, the first protease is a variant of SEQ ID NO: 1 comprising the substitutions K27M+N62D+G97D+Y209W+A215K+L262E, i.e. having a net formal charge of -3 relative to SEQ ID NO: 1.

In one embodiment, the first protease is a variant of SEQ ID NO: 1 comprising the substitutions S9E+G97D+S101E+Y209W+A215K+L262E, i.e. having a net formal charge of -3 relative to SEQ ID NO: 1.

In one embodiment, the first protease is a variant of SEQ ID NO: 1 comprising the substitutions S9E+N76D+G97D+Y209W+A215K+L262E+A270T, i.e. having a net formal charge of -3 relative to SEQ ID NO: 1.

In one embodiment, the first protease is a variant of SEQ ID NO: 1 comprising the substitutions S9E+N76D+G97D+Y209W+A215K+L262E, i.e. having a net formal charge of -3 relative to SEQ ID NO: 1.

In one embodiment, the first protease is a variant of SEQ ID NO: 1 comprising the substitutions S9E+N62D+G97D+Y209W+A215K+L262E, i.e. having a net formal charge of -3 relative to SEQ ID NO: 1.

In one embodiment, the first protease is a variant of SEQ ID NO: 1 comprising the substitutions S9E+K27M+G97-

D+Y209W+A215K+L262E, i.e. having a net formal charge of -3 relative to SEQ ID NO: 1.

In one embodiment, the first protease is a variant of SEQ ID NO: 1 comprising the substitutions S9E+N43-R+N76D+N185E+S188E+Q191N+A194P+Q206L+Y209W+A215K+ Q245R+S259D+L262E, i.e. having a net formal charge of -3 relative to SEQ ID NO: 1.

In one embodiment, the first protease is a variant of SEQ ID NO: 1 comprising the substitutions S9E+N43-R+N76D+S188E+Q191N+A194P+Q206L+Y209W+A215K+S259D+ L262E, i.e. having a net formal charge of -3 relative to SEQ ID NO: 1.

In one embodiment, the first protease is a variant of SEQ ID NO: 1 comprising the substitutions S9E+N43-R+N76D+N185E+S188E+Q191N+A194P+Q206L+Y209W+A215K+ S259D+L262Q, i.e. having a net formal charge of -3 relative to SEQ ID NO: 1.

In one embodiment, the first protease is a variant of SEQ ID NO: 1 comprising the substitutions S9E+N43-R+N76D+N185E+S188E+Q191N+A194P+Q206L+Y209W+Q245R+ S259D+L262Q, i.e. having a net formal charge of -3 relative to SEQ ID NO: 1.

In one embodiment, the second protease (or first protease, where the second protease has a net formal charge of -2) is a variant of SEQ ID NO: 1 comprising the substitution K27M, i.e. having a net formal charge of -1 relative to SEQ ID NO: 1.

In one embodiment, the second protease is the polypeptide of SEQ ID NO: 1.

In one embodiment, the second protease (or first protease, where the second protease has a net formal charge of -2) is a variant of SEQ ID NO: 1 comprising the mutation S99AD (i.e. substitution of S to A, and insertion of D), i.e. having a net formal charge of -1 relative to SEQ ID NO: 1.

In one embodiment, the second protease is a variant of SEQ ID NO: 1 comprising the substitutions G97D+Y209-W+A215K, i.e. having a net formal charge of 0 relative to SEQ ID NO: 1.

In one embodiment, the second protease is a variant of SEQ ID NO: 1 comprising the substitutions G97D+N117R+Y209W+A215K, i.e. having a net formal charge of +1 relative to SEQ ID NO: 1.

In one embodiment, the second protease is a variant of SEQ ID NO: 1 comprising the substitutions S9R+K27M+N43R+N76D+V205I+Q206L+Y209W+A215K+Q245R+S259D+ N261W+L262E, i.e. having a net formal charge of 0 relative to SEQ ID NO: 1.

In one embodiment, the second protease is a variant of SEQ ID NO: 1 comprising the substitutions S9R+N43R+N76D+V205I+Q206L+Y209W+A215K+S259D+N261W+L262E, i.e. having a net formal charge of 0 relative to SEQ ID NO: 1.

In one embodiment, the second protease (or first protease, where the second protease has a net formal charge of -2) is a variant of SEQ ID NO: 1 comprising the substitutions S9R+N43R+N76D+V205I+Q206-L+Y209W+S259D+N261W+L262E, i.e. having a net formal charge of-1 relative to SEQ ID NO: 1.

In one embodiment, the second protease (or first protease, where the second protease has a net formal charge of -2) is a variant of SEQ ID NO: 1 comprising the substitutions S9R+S99A+S101E+S103A+V104I+S156D+G160-S+A215K+K235M+Q245R+L262E, i.e. having a net formal charge of -1 relative to SEQ ID NO: 1.

In one embodiment, the second protease (or first protease, where the second protease has a net formal charge of -2) is a variant of SEQ ID NO: 1 comprising the substitutions S9R+K27M+N43R+S99A+S101E+S103A+V104I+S156D+G160S+A215K+K237M+Q245R+ L262E, i.e. having a net formal charge of -1 relative to SEQ ID NO: 1.

In one embodiment, the second protease (or first protease, where the second protease has a net formal charge of -2) is a variant of SEQ ID NO: 1 comprising the substitutions S9R+K27M+N43R+S101E+S103A+V104I+S156D+G160-S+A215K+K237M+Q245R+L262E, i.e. having a net formal charge of -1 relative to SEQ ID NO: 1.

In one embodiment, the second protease (or first protease, where the second protease has a net formal charge of -2) is a variant of SEQ ID NO: 1 comprising the substitutions Y167A+R170S+A194P, i.e. having a net formal charge of -1 relative to SEQ ID NO: 1.

In one embodiment, the second protease is a variant of SEQ ID NO: 1 comprising the substitutions S9R+A15T+V68A+N218D+Q245R, i.e. having a net formal charge of +1 relative to SEQ ID NO: 1.

In one embodiment, the second protease is a variant of SEQ ID NO: 1 comprising the substitutions S9R+A15T+G61E+V68A+A194P+V205I+Q245R+N261D, i.e. having a net formal charge of 0 relative to SEQ ID NO: 1.

In one embodiment, the second protease is a variant of SEQ ID NO: 1 comprising the substitutions S9R+A15T+V68A+S99G+Q245R+N261D, i.e. having a net formal charge of +1 relative to SEQ ID NO: 1.

In one embodiment, the second protease is a variant of SEQ ID NO: 1 comprising the substitutions S9R+A15T+V68A+H120D+P131S+Q137H+Q245R, i.e. having a net formal charge of +1 relative to SEQ ID NO: 1.

In one embodiment, the second protease is a variant of SEQ ID NO: 1 comprising the substitutions S9R+A15T+V68A+H120N+P131S+Q137H+Q245M, i.e. having a net formal charge of +1 relative to SEQ ID NO: 1.

In one embodiment, the second protease is a variant of SEQ ID NO: 1 comprising the substitutions S9R+A15T+G61E+V68A+A98S+S99G+N218D+Q245R, i.e. having a net formal charge of 0 relative to SEQ ID NO: 1.

In one embodiment, the second protease (or first protease, where the second protease has a net formal charge of -2) is a variant of SEQ ID NO: 1 comprising the substitutions S3T+V4I+S99D+S101R+S103A+V104I+G160S+V205I+L217D, i.e. having a net formal charge of-1 relative to SEQ ID NO: 1.

In one embodiment, the second protease (or first protease, where the second protease has a net formal charge of -2) is a variant of SEQ ID NO: 1 comprising the substitutions S3T+V4I+S99D+S101R+S103A+V104I+G160-S+A194P+V205I+L217D, i.e. having a net formal charge of-1 relative to SEQ ID NO: 1.

In one embodiment, the second protease (or first protease, where the second protease has a net formal charge of -2) is a variant of SEQ ID NO: 1 comprising the substitutions G97D+Y209W+A215K+L262E, i.e. having a net formal charge of -1 relative to SEQ ID NO: 1.

In one embodiment, the second protease (or first protease, where the second protease has a net formal charge of -2) is a variant of SEQ ID NO: 1 comprising the substitutions G97D+S156D+Y209W+A215K, i.e. having a net formal charge of -1 relative to SEQ ID NO: 1.

Other suitable protease variants that may be used as the first or second protease are those that are at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to any of the proteases disclosed above, and where any additional mutations in the individual protease variants as set forth above result in the same net formal charge.

Exemplary, non-limiting embodiments of the invention with specific first and second proteases are provided in the following.

In one embodiment, the first protease is a variant of SEQ ID NO: 1 comprising the substitutions S99D+S101E+S103A+V104I+S156D+G160S+L262E, and the second protease is a variant of SEQ ID NO: 1 comprising the substitution K27M.

In one embodiment, the first protease is a variant of SEQ ID NO: 1 comprising the substitutions K27H+S99D+S101E+S103A+V104I+S156D+G160S+Q245R+L262E, and the second protease is a variant of SEQ ID NO: 1 comprising the substitution K27M.

In one embodiment, the first protease is a variant of SEQ ID NO: 1 comprising the substitutions S9R+S99D+S101E+S103A+V104I+S156D+G160S+K235M+Q245R+L262E, and the second protease is a variant of SEQ ID NO: 1 comprising the substitution K27M.

In one embodiment, the first protease is a variant of SEQ ID NO: 1 comprising the substitutions S9R+K27M+S99D+S101E+S103A+V104I+S156D+G160S+K237M+Q245R+ L262E, and the second protease is a variant of SEQ ID NO: 1 comprising the substitution K27M.

In one embodiment, the first protease is a variant of SEQ ID NO: 1 comprising the substitutions S99D+S101E+S103A+V104I+S156D+G160S+L262E, and the second protease is the polypeptide of SEQ ID NO: 1.

In one embodiment, the first protease is a variant of SEQ ID NO: 1 comprising the substitutions K27H+S99D+S101E+S103A+V104I+S156D+G160S+Q245R+L262E, and the second protease is the polypeptide of SEQ ID NO: 1.

In one embodiment, the first protease is a variant of SEQ ID NO: 1 comprising the substitutions S9R+K27M+S99D+S101E+S103A+V104I+S156D+G160S+K237M+Q245R+ L262E, and the second protease is the polypeptide of SEQ ID NO: 1.

In one embodiment, the first protease is a variant of SEQ ID NO: 1 comprising the substitutions S99D+S101E+S103A+V104I+S156D+G160S+L262E, and the second protease is a variant of SEQ ID NO: 1 comprising the mutation S99AD.

In one embodiment, the first protease is a variant of SEQ ID NO: 1 comprising the substitutions K27H+S99D+S101E+S103A+V104I+S156D+G160S+Q245R+L262E, and the second protease is a variant of SEQ ID NO: 1 comprising the mutation S99AD.

In one embodiment, the first protease is a variant of SEQ ID NO: 1 comprising the substitutions S9R+S99D+S101E+S103A+V104I+S156D+G160S+K235M+Q245R+L262E, and the second protease is a variant of SEQ ID NO: 1 comprising the mutation S99AD.

In one embodiment, the first protease is a variant of SEQ ID NO: 1 comprising the substitutions S9R+S99D+S101E+S103A+V104I+S156D+G160S+L262E, and the second protease is a variant of SEQ ID NO: 1 comprising the mutation S99AD.

In one embodiment, the first protease is a variant of SEQ ID NO: 1 comprising the substitutions S9R+K27M+S99D+S101E+S103A+V104I+S156D+G160S+K237M+Q245R+ L262E, and the second protease is a variant of SEQ ID NO: 1 comprising the mutation S99AD.

In one embodiment, the first protease is a variant of SEQ ID NO: 1 comprising the substitutions S99D+S101E+S103A+V104I+S156D+G160S+L262E, and the second protease is a variant of SEQ ID NO: 1 comprising the substitutions G97D+Y209W+A215K or G97D+N 117R+Y209W+A215K.

In one embodiment, the first protease is a variant of SEQ ID NO: 1 comprising the substitutions S9E+N43-R+N76D+V205I+Q206L+Y209W+S259D+N261W+L262E, and the second protease is a variant of SEQ ID NO: 1 comprising the substitutions G97D+Y209W+A215K or G97D+N117R+Y209W+A215K.

In one embodiment, the first protease is a variant of SEQ ID NO: 1 comprising the substitutions S9E+N43-R+N76D+N185E+S188E+Q191N+A194P+Q206L+Y209W+S259D+ L262E, and the second protease is a variant of SEQ ID NO: 1 comprising the substitutions G97D+Y209W+A215K or G97D+N117R+Y209W+A215K.

In one embodiment, the first protease is a variant of SEQ ID NO: 1 comprising the mutations *36D+N76D+H120D+G195E+K235L, and the second protease is a variant of SEQ ID NO: 1 comprising the substitutions G97D+Y209W+A215K or G97D+N117R+Y209W+A215K.

In one embodiment, the first protease is a variant of SEQ ID NO: 1 comprising the substitution K27M, and the second protease is a variant of SEQ ID NO: 1 comprising the substitutions S9R+S99D+S101E+S103A+V104I+S156D+G160S+Q245R+L262E.

In one embodiment, the first protease is the polypeptide of SEQ ID NO: 1, and the second protease is a variant of SEQ ID NO: 1 comprising the substitutions S9R+S99D+S101E+ S103A+V104I+S156D+G160S+Q245R+L262E.

In one embodiment, the first protease is a variant of SEQ ID NO: 1 comprising the mutation S99AD, and the second protease is a variant of SEQ ID NO: 1 comprising the substitutions S9R+S99D+S101E+S103A+V104I+S156D+G160S+Q245R+L262E.

In one embodiment, the first protease is a variant of SEQ ID NO: 1 comprising the substitutions N76D+G97D+N140D+S156D+Y209W+A215K+L262E, and the second protease is the polypeptide of SEQ ID NO: 1.

In one embodiment, the first protease is a variant of SEQ ID NO: 1 comprising the substitutions N76D+G97D+N140D+S156D+Y209W+A215K+L262E, and the second protease is a variant of SEQ ID NO: 1 comprising the substitutions G97D+S156D+Y209W+A215K.

In one embodiment, the first protease is a variant of SEQ ID NO: 1 comprising the substitutions N76D+G97D+N140D+S156D+Y209W+A215K+L262E, and the second protease is a variant of SEQ ID NO: 1 comprising the substitutions G97D+Y209W+A215K+L262E.

In one embodiment, the first protease is a variant of SEQ ID NO: 1 comprising the substitutions K27M+N77D+G97D+S156D+Y209W+A215K+L262E, and the second protease is the polypeptide of SEQ ID NO: 1.

In one embodiment, the first protease is a variant of SEQ ID NO: 1 comprising the substitutions K27M+N77D+G97D+S156D+Y209W+A215K+L262E, and the second protease is a variant of SEQ ID NO: 1 comprising the substitutions G97D+S156D+Y209W+A215K.

In one embodiment, the first protease is a variant of SEQ ID NO: 1 comprising the substitutions K27M+N77D+G97D+S156D+Y209W+A215K+L262E, and the second protease is a variant of SEQ ID NO: 1 comprising the substitutions G97D+Y209W+A215K+L262E.

In one embodiment, the first protease is a variant of SEQ ID NO: 1 comprising the substitutions G97D+N117R+Y209W+A215K, and the second protease is a variant of SEQ ID NO: 1 comprising the substitutions G97D+S156D+Y209W+A215K+L262E.

In one embodiment, the first protease is a variant of SEQ ID NO: 1 comprising the substitutions G97D+S156D+Y209W+A215K, and the second protease is a variant of SEQ ID NO: 1 comprising the substitutions G97D+S156D+Y209W+A215K+L262E.

In one embodiment, the first protease is a variant of SEQ ID NO: 1 comprising the substitutions G97D+Y209-W+A215K+L262E, and the second protease is a variant of SEQ ID NO: 1 comprising the substitutions G97D+S156D+Y209W+A215K+L262E.

In one embodiment, the first protease is a variant of SEQ ID NO: 1 comprising the substitutions N62D+N76D+G97D+Y209W+A215K+L262E, and the second protease is a variant of SEQ ID NO: 1 comprising the substitutions G97D+S156D+Y209W+A215K+L262E.

In one embodiment, the first protease is a variant of SEQ ID NO: 1 comprising the substitutions N62D+G97D+S101E+V177I+Y209W+A215K+L262E, and the second protease is a variant of SEQ ID NO: 1 comprising the substitutions G97D+S156D+Y209W+A215K+L262E.

In one embodiment, the first protease is a variant of SEQ ID NO: 1 comprising the substitutions N62D+G97D+S101E+Y209W+A215K+L262E, and the second protease is a variant of SEQ ID NO: 1 comprising the substitutions G97D+S156D+Y209W+A215K+L262E.

In one embodiment, the first protease is a variant of SEQ ID NO: 1 comprising the substitutions G97D+S101E+S156D+A172V+Y209W+A215K+L262E, and the second protease is a variant of SEQ ID NO: 1 comprising the substitutions G97D+S156D+Y209W+A215K+L262E.

In one embodiment, the first protease is a variant of SEQ ID NO: 1 comprising the substitutions N76D+G97D+N140D+S156D+Y209W+A215K+L262E, and the second protease is a variant of SEQ ID NO: 1 comprising the substitutions G97D+S156D+Y209W+A215K+L262E.

In one embodiment, the first protease is a variant of SEQ ID NO: 1 comprising the substitutions

K27M+N77D+G97D+S156D+Y209W+A215K+L262E, and the second protease is a variant of SEQ ID NO: 1 comprising the substitutions G97D+S156D+Y209W+A215K+L262E.

In one embodiment, the first protease has a net formal charge of -1, -3 or -4 relative to the protease of SEQ ID NO: 1, and the second protease has a net formal charge of -2 relative to the protease of SEQ ID NO: 1 and is a variant of SEQ ID NO: 1 with the substitutions G97D+S156D+Y209W+A215K+L262E or S9R+S99D+S101E+S103A+V104I+S156D+G160S+ Q245R+L262E, preferably G97D+S156D+Y209W+A215-K+L262E.

In one embodiment, the first protease has a net formal charge of -1, -3 or -4 relative to the protease of SEQ ID NO: 1, and the second protease has a net formal charge of -2 relative to the protease of SEQ ID NO: 1, wherein the first protease is a variant of SEQ ID NO: 1 having a set of mutations selected from the group consisting of:

- K27M;
- S99AD;
- G97D+S156D+Y209W+A215K;
- G97D+Y209W+A215K+L262E;
- S9R+S99D+S101E+S103A+V104I+S156D+G160S+K235M+Q245R+L262E;
- S9R+S99D+S101E+S103A+V104I+S156D+G160S+L262E;
- S9E+N43R+N76D+V205I+Q206L+Y209W+S259D+N261W+L262E;
- N62D+N76D+G97D+Y209W+A215K+L262E;
- N62D+G97D+S101E+V177I+Y209W+A215K+L262E;
- N62D+G97D+S101E+Y209W+A215K+L262E;
- G97D+S101E+S156D+A172V+Y209W+A215K+L262E;
- S99D+S101E+S103A+V104I+S156D+G160S+L262E;
- K27H+S99D+S101E+S103A+V104I+S156D+G160S+Q245R+L262E;
- S9R+K27M+S99D+S101E+S103A+V104I+S156D+G160S+K237M+Q245R+L262E;
- N76D+G97D+N140D+S156D+Y209W+A215K+L262E; and
- K27M+N77D+G97D+S156D+Y209W+A215K+L262E;

and where the second protease preferably is a variant of SEQ ID NO: 1 with the substitutions G97D+S156D+Y209-W+A215K+L262E or S9R+S99D+S101E+S103A+V104I+S156D+G160S+ Q245R+L262E, preferably G97D+S156D+Y209W+A215K+L262E.

In one embodiment, the first protease has a net formal charge of -1, -3 or -4 relative to the protease of SEQ ID NO: 1, and the second protease has a net formal charge of -2 relative to the protease of SEQ ID NO: 1, wherein the second protease is a variant of SEQ ID NO: 1 having a set of mutations selected from the group consisting of:

- S87N+G97D+S156D+Y209W+A215K+L262E;
- G97D+S101G+S156D+Y209W+A215K+L262E;
- G97D+V104N+S156D+Y209W+A215K+L262E;
- G97D+G118V+S156D+Y209W+A215K+L262E;
- G97D+S156D+A194P+Y209W+A215K+L262E;
- S87N+G97D+S101G+S156D+Y209W+A215K+L262E;
- S87N+G97D+S101N+S156D+Y209W+A215K+L262E;
- S87N+G97D+V104N+S156D+Y209W+A215K+L262E;
- S87N+G97D+G118V+S156D+Y209W+A215K+L262E;
- S87N+G97D+S156D+A194P+Y209W+A215K+L262E;
- G97D+S101G+G118V+S156D+Y209W+A215K+L262E;
- G97D+S101G+S156D+A194P+Y209W+A215K+L262E; and
- G97D+S101N+V104N+S156D+Y209W+A215K+L262E.

When the first protease has a net formal charge of -3, -4 or -5 relative to the protease of SEQ ID NO: 1 and a second protease has a net formal charge of -1, 0 or +1 relative to the protease of SEQ ID NO: 1, the first protease may be a variant of SEQ ID NO: 1 comprising a set of mutations selected from the group consisting of:

- S99D+S101E+S103A+V104I+S156D+G160S+L262E;
- K27H+S99D+S101E+S103A+V104I+S156D+G160S+Q245R+L262E;
- S9R+S99D+S101E+S103A+V104I+S156D+G160S+K235M+Q245R+L262E;
- S9R+S99D+S101E+S103A+V104I+S156D+G160S+L262E;
- S9R+K27M+S99D+S101E+S103A+V104I+S156D+G160S+K237M+Q245R+L262E;

- S9E+N43R+N76D+V205I+Q206L+Y209W+S259D+N261W+L262E;
- S9E+N43R+N76D+N185E+S188E+Q191N+A194P+Q206L+Y209W+S259D+L262E;
- *36D+N76D+H120D+G195E+K235L;
- S9R+K27M+S99A+S101E+S103A+V104I+S156D+G160S+K237M+Q245R+L262E;
- S9R+K27M+S101E+S103A+V104I+S156D+G160S+K237M+Q245R+L262E;
- S9R+K27M+S99D+S101E+S103A+V104I+S156D+G160S+A215K+K237M+Q245R+ L262E;
- S9R+K27M+N43R+S99D+S101E+S103A+V104I+S156D+G160S+K237M+Q245R+ L262E;
- S9R+N43R+S99D+S101E+S103A+V104I+S156D+G160S+N173D+K235M+Q245R +L262E;
- S9E+G97D+S156D+Y209W+A215K+L262E;
- G97D+S101E+S156D+A172V+Y209W+A215K+L262E;
- N76D+G97D+N140D+S156D+Y209W+A215K+L262E;
- N76D+G97D+S101E+T180A+Y209W+A215K+L262E;
- N76D+G97D+S101E+Y209W+A215K+L262E;
- N62D+G97D+S101E+Y209W+A215K+L262E;
- N62D+G97D+S101E+V177I+Y209W+A215K+L262E;
- N62D+N76D+G97D+Y209W+A215K+L262E;
- K27M+G97D+S156D+Y209W+A215K+L262E;
- K27M+N77D+G97D+S156D+Y209W+A215K+L262E;
- K27M+N76D+G97D+Y209W+A215K+L262E;
- K27M+N62D+G97D+Y209W+A215K+L262E;
- S9E+G97D+S101E+Y209W+A215K+L262E;
- S9E+N76D+G97D+Y209W+A215K+L262E+A270T;
- S9E+N76D+G97D+Y209W+A215K+L262E;
- S9E+N62D+G97D+Y209W+A215K+L262E;
- S9E+K27M+G97D+Y209W+A215K+L262E;
- S9E+N43R+N76D+N185E+S188E+Q191N+A194P+Q206L+Y209W+A215K+Q245R +S259D+L262E;
- S9E+N43R+N76D+S188E+Q191N+A194P+Q206L+Y209W+A215K+S259D+L262E;
- S9E+N43R+N76D+N185E+S188E+Q191N+A194P+Q206L+Y209W+A215K+S259D +L262Q; and
- S9E+N43R+N76D+N185E+S188E+Q191N+A194P+Q206L+Y209W+Q245R+S259D +L262Q;

and the second protease may be the protease of SEQ ID NO: 1 or a variant of SEQ ID NO: 1 comprising a set of mutations selected from the group consisting of:

- K27M;
- S99AD;
- G97D+Y209W+A215K;
- G97D+N117R+Y209W+A215K;
- S9R+K27M+N43R+N76D+V205I+Q206L+Y209W+A215K+Q245R+S259D+N261W+ L262E;
- S9R+N43R+N76D+V205I+Q206L+Y209W+A215K+S259D+N261W+L262E;
- S9R+N43R+N76D+V205I+Q206L+Y209W+S259D+N261W+L262E;
- S9R+S99A+S101E+S103A+V104I+S156D+G160S+A215K+K235M+Q245R+L262E;
- S9R+K27M+N43R+S99A+S101E+S103A+V104I+S156D+G160S+A215K+K237M+ Q245R+L262E;
- S9R+K27M+N43R+S101E+S103A+V104I+S156D+G160S+A215K+K237M+Q245R +L262E;
- Y167A+R170S+A194P;
- S9R+A15T+V68A+N218D+Q245R;
- S9R+A15T+G61E+V68A+A194P+V205I+Q245R+N261D;
- S9R+A15T+V68A+S99G+Q245R+N261D;
- S9R+A15T+V68A+H120D+P131S+Q137H+Q245R;
- S9R+A15T+V68A+H120N+P131S+Q137H+Q245M;
- S9R+A15T+G61E+V68A+A98S+S99G+N218D+Q245R;
- S3T+V4I+S99D+S101R+S103A+V104I+G160S+V205I+L217D;
- S3T+V4I+S99D+S101R+S103A+V104I+G160S+A194P+V205I+L217D;
- G97D+Y209W+A215K+L262E; and
- G97D+S156D+Y209W+A215K.

In some preferred embodiments, where the first protease has a net formal charge of -3, -4 or -5 relative to the protease of SEQ ID NO: 1 and the second protease has a net formal charge of -1, 0 or +1 relative to the protease of SEQ ID NO: 1, the first protease may be a variant of SEQ ID NO: 1 having a set of mutations selected from the group consisting of:

- S9E+N43R+N76D+N185E+S188E+Q191N+A194P+Q206L+Y209W+S259D+L262E;
- *36D+N76D+H120D+G195E+K235L;
- S99D+S101E+S103A+V104I+S156D+G160S+L262E;
- K27H+S99D+S101E+S103A+V104I+S156D+G160S+Q245R+L262E;
- S9R+K27M+S99D+S101E+S103A+V104I+S156D+G160S+K237M+Q245R+L262E;
- N76D+G97D+N140D+S156D+Y209W+A215K+L262E;
- K27M+N77D+G97D+S156D+Y209W+A215K+L262E;
- S9R+S99D+S101E+S103A+V104I+S156D+G160S+K235M+Q245R+L262E;
- S9R+S99D+S101E+S103A+V104I+S156D+G160S+L262E;
- S9E+N43R+N76D+V205I+Q206L+Y209W+S259D+N261W+L262E;
- N62D+N76D+G97D+Y209W+A215K+L262E;
- N62D+G97D+S101E+V177I+Y209W+A215K+L262E;
- N62D+G97D+S101E+Y209W+A215K+L262E; and
- G97D+S101E+S156D+A172V+Y209W+A215K+L262E;

and the second protease may be the protease of SEQ ID NO: 1 or a variant thereof having a set of mutations selected from the group consisting of:

- K27M;
- S99AD;
- G97D+S156D+Y209W+A215K;
- G97D+Y209W+A215K+L262E;
- G97D+Y209W+A215K; and
- G97D+N117R+Y209W+A215K.

In the aspect of the invention where the first protease has a net formal charge of 0, -1 or -2 relative to the protease of SEQ ID NO: 1, and the second protease has a net formal charge of +2, +3, +4 or +5 relative to the protease of SEQ ID NO: 1, the first protease having a net formal charge of 0, -1 or -2 may, for example, be selected from the polypeptide of SEQ ID NO: 1 and variants of SEQ ID NO: 1 having a set of mutations selected from the group consisting of:

- G97D+Y209W+A215K
- S9R+K27M+N43R+N76D+V205I+Q206L+Y209W+A215K+Q245R+S259D+ N261W+L262E
- S9R+N43R+N76D+V205I+Q206L+Y209W+A215K+S259D+N261W+L262E
- S9R+A15T+G61E+V68A+A194P+V205I+Q245R+N261D
- S9R+A15T+G61E+V68A+A98S+S99G+N218D+Q245R
- K27M
- S99AD
- S9R+N43R+N76D+V205I+Q206L+Y209W+S259D+N261W+L262E
- S9R+S99A+S101E+S103A+V104I+S156D+G160S+A215K+K235M+Q245R+L262E
- S9R+K27M+N43R+S99A+S101E+S103A+V104I+S156D+G160S+A215K+K237M +Q245R+L262E
- S9R+K27M+N43R+S101E+S103A+V104I+S156D+G160S+A215K+K237M+Q245R +L262E
- Y167A+R170S+A194P
- S3T+V4I+S99D+S101R+S103A+V104I+G160S+V205I+L217D
- S3T+V4I+S99D+S101R+S103A+V104I+G160S+A194P+V205I+L217D
- G97D+Y209W+A215K+L262E
- G97D+S156D+Y209W+A215K
- S9R+S99D+S101E+S103A+V104I+S156D+G160S+Q245R+L262E;
- S9R+K27M+N43R+S101E+S103A+V104I+S156D+G160S+K237M+Q245R+L262E;
- S9R+K27M+N43R+S99D+S101E+S103A+V104I+S156D+G160S+A215K+K237M+ Q245R+L262E;
- S9R+S99A+S101E+S103A+V104I+A151V+S156D+G160S+K235M+Q245R+L262E;
- S9R+S99A+S101E+S103A+V104I+S156D+G160S+K235M+Q245R+L262E;
- S9R+S990+S101E+S103A+V104I+S156D+G160S+A215K+K235M+Q245R+L262E;
- N62D+G97D+Y209W+A215K+L262E;
- G97D+S156D+Y209W+A215K+L262E;
- G97D+S101E+Y209W+A215K+L262E;
- N76D+G97D+Y209W+A215K+L262E;
- K27M+G97D+Y209W+A215K+L262E;
- S9E+G97D+Y209W+A215K+L262E;

- S9R+N43R+N76D+N185E+Q191N+A194P+Q206L+Y209W+S259D+L262E; and
- S9E+N43R+N76D+V205I+Q206L+Y209W+Q245R+S259D+N261W+L262E.

In an additional aspect, the first protease may have a net formal charge of -2 relative to SEQ ID NO: 1, and the second protease may have a net formal charge of -1, 0 or +1 relative to SEQ ID NO: 1.

As an example of an embodiment of this aspect, the first protease may be a variant of SEQ ID NO: 1 comprising the substitutions S9R+S99D+S101E+S103A+V104I+S156D+ G160S+Q245R+L262E, and the second protease may be the polypeptide of SEQ ID NO: 1 or a variant of SEQ ID NO: 1 comprising the substitution K27M or comprising the mutation S99AD.

In one embodiment of the invention, the detergent composition of the invention does not comprise two proteases wherein one protease is comprised in a solid enzyme formulation and the other protease is comprised in a liquid enzyme formulation.

In one embodiment, the detergent composition of the invention does not comprise the protease of SEQ ID NO: 8 disclosed in US 2017/0198243. In one embodiment, the detergent composition of the invention does not comprise the protease of SEQ ID NO: 9 disclosed in US 2017/0198243. In a further embodiment, the two proteases in the detergent composition of the invention are not the protease of SEQ ID NO: 8 and the protease of SEQ ID NO: 9 disclosed in US 2017/0198243.

Preferably, the detergent composition of the invention comprising a first protease and a second protease as defined above has an improved wash performance compared to the same composition comprising either the first protease or the second protease. The improved wash performance may be improved performance on one or more individual stains and/or improved overall performance on a set of stains, e.g. 2, 3, 4, 5 or more stains. Wash performance may e.g. be determined using the AMSA method described herein, for example using the model detergent set forth in Table 1. Performance may be tested on any suitable stain(s) for the intended application, e.g. laundry or automatic dishwashing. Examples of such stains are e.g. the standard laundry stains PC-10 and/or PC-03.

Proteases with a relatively negative net formal charge of e.g. -4 or -3 compared to SEQ ID NO: 1 tend to be better at removing certain types of protein stains, for example cocoa-containing stains. On the other hand, proteases with a more positive, relatively neutral net formal charge of e.g. -1 or 0 tend to be better at removing other types of protein stains, for example sebum stains and other oil-containing protein stains. It was therefore surprising that the detergent compositions of the invention comprising two proteases with different net formal charge characteristics could provide improved cleaning of different types of stains without increasing the total amount of protease enzyme.

It was also surprising that it was possible to obtain significantly better removal of the oily PC-10 stain using a mixture of the two different proteases with different charge characteristics rather than relying on a relatively neutral protease that is otherwise better at removing oil-containing protein stains. The PC-10 stain (pigment, oil, milk) mimics natural sebum stains that contain both protein and oil/fat, thus providing a good indication of how a detergent composition will perform on this difficult to remove natural stain.

In one embodiment, the detergent composition of the invention comprising a first protease and a second protease has an improved wash performance on PC-10 compared to the same composition comprising either the first protease or the second protease.

In another embodiment, the detergent composition of the invention comprising a first protease and a second protease has an improved overall wash performance on PC-10 and PC-03 compared to the same composition comprising either the first protease or the second protease.

In one embodiment, the relative wash performance of the detergent composition of the invention comprising a first protease and a second protease is improved over the wash performance of a composition comprising either the first protease or the second protease by at least 1%, preferably at least 2%, at least 3%, at least 4% or at least 5%, such as at least 6%, at least 7%, at least 8%, at least 9% or at least 10%, e.g. at least 15% or at least 20%. This improved relative wash performance is preferably obtained at least on the PC-10 stain from Center for Testmaterials (CFT). More preferably, this improved relative wash performance is an improved overall wash performance on a set of stains comprising PC-10 and PC-03 (both from CFT), and optionally comprising one or more additional standard stains. Examples of such additional stains that may be used are one or more of C-S-05S, C-S-67, C-S-75, C-S-95, C-H151, C-H163, C-H252, C-S-60, all from Center for Testmaterials (CFT), and French Mustard WE5FSMWKC, 011 KC WC PC PE Bacon Grease, 028 KC WC PC PE Cheese Spread, 080 KC WC PC PE Mayonnaise, all from Warwick Equest. Relative wash performance may e.g. be determined based on the sums of delta intensity as described in Example 1.

It will be understood that when comparing the wash performance of a composition of the invention with a corresponding composition comprising only a single enzyme, i.e. either the first protease or the second protease, the compositions being compared will contain the same total amount of protease enzyme protein (by weight).

In a further aspect, the invention relates to novel protease variants with advantageous properties, where the proteases are variants of SEQ ID NO: 1 comprising substitutions in positions 97, 209 and 215. Such proteases have been found to not only have beneficial properties when used in detergent compositions with another protease having a different

net formal charge such as those disclosed herein, but also to have beneficial properties, e.g. improved laundry wash performance, when used alone.

In this aspect the invention relates to a protease variant comprising the substitutions X97D, X209W and X215K, e.g. G97D, Y209W and A215K, wherein position numbers are based on the numbering of SEQ ID NO: 2, and the variant has protease activity and has at least 80%, such as at least 85%, at least 90% or at least 95%, but less than 100% sequence identity to SEQ ID NO: 1. In some embodiments of this aspect of the invention, the protease variant comprises one or more additional substitutions, preferably in one or more positions, for example in one, two, three or four positions, selected from positions 27, 62, 76, 77, 87, 101, 104, 117, 118, 140, 156, 172, 177, 194 and 262. Such additional substitutions include, in particular, X9E (e.g. S9E), X27M (e.g. K27M), X62D (e.g. N62D), X76D (e.g. N76D), X77D (e.g. N77D), X87N (e.g. S87N), X101E/G/N (e.g. S101E/G/N), X104N (e.g. V104N), X117R (e.g. N117R), X118V (e.g. G118V), X140D (e.g. N140D), X156D (e.g. S156D), X172V (e.g. A172V), X177I (e.g. V177I), X194P (e.g. A194P) and X262E (e.g. L262E). Particular embodiments of this aspect of the invention are provided below.

In one embodiment, the protease is a variant of SEQ ID NO: 1 comprising the substitutions G97D+Y209-W+A215K, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S156D+Y209-W+A215K, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+Y209-W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+N117R+Y209-W+A215K, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S156D+Y209-W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions K27M+N77D+G97D+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions N62D+N76D+G97D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions N62D+G97D+S101E+V177I+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions N62D+G97D+S101E+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions N76D+G97D+N140D+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S101E+S156-

D+A172V+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S9E+N62D+G97D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions K27M+G97D+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S9E+G97D+S156-D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions N62D+G97D+Y209-W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1.

In one embodiment, the protease is a variant of SEQ ID NO: 1 comprising the substitutions N76D+G97D+S101E+T180A+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions N76D+G97D+S101E+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions K27M+N76D+G97D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions K27M+N62D+G97D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S9E+G97D+S101E+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S9E+N76D+G97D+Y209W+A215K+L262E+A270T, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S9E+N76D+G97D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S9E+K27M+G97-

D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S101E+Y209-W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions N76D+G97D+Y209-W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions K27M+G97D+Y209-W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S9E+G97D+Y209-W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one aspect, the invention relates to a protease which is a variant of SEQ ID NO: 1 comprising the substitutions G97D+S156D+Y209W+A215K+L262E and optionally at least one additional substitution selected from S87N, S101G/N, V104N, G118V and A194P. Examples of such proteases are provided below.

In one embodiment, the protease is a variant of SEQ ID NO: 1 comprising the substitutions S87N+G97D+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S101G+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S101N+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+V104N+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+G118V+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S156D+A194-P+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S87N+G97D+S101G+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S87N+G97D+S101N+S156D+Y209W+A215K+L262E, wherein position numbers are based on the numbering of

SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S87N+G97D+V104N+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S87N+G97D+G118V+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions S87N+G97D+S156D+A194P+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S101G+G118V+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S101G+S156D+A194P+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

In one embodiment, the protease is a variant of SEQ ID NO: 1 comprising the substitutions G97D+S101N+V104N+S156D+Y209W+A215K+L262E, wherein position numbers are based on the numbering of SEQ ID NO: 2, and the variant has protease activity and has at least 80%, such as at least 85%, at least 90% or at least 95%, but less than 100% sequence identity to SEQ ID NO: 1. The protease may e.g. comprise or consist of SEQ ID NO: 1 with this set of substitutions.

It will be apparent from the description above and the examples below that in the detergent compositions of the invention comprising two proteases with different net charge characteristics, one or both of the proteases may be a variant of SEQ ID NO: 1 comprising the substitutions G97D+Y209W+A215K, and optionally other substitutions as set forth herein, as long as the two proteases fulfill the relevant net formal charge criteria relative to SEQ ID NO: 1.

In addition to the amino acid alterations specifically disclosed herein, a protease variant in a composition of the invention may comprise additional alterations at one or more other positions. These additional alterations may be of a minor nature, that is typically conservative amino acid substitutions or insertions that do not significantly affect the folding and/or activity of the protein, and which do not alter the net formal charge as described herein; small deletions, typically of 1-30 amino acids; or small amino- or carboxyl-terminal extensions.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, in The Proteins, Academic Press, New York. Common conservative substitution groups include, but are not limited to: G=A=S; I=V=L=M; D=E; Y=F; and N=Q (where e.g. "G=A=S" means that these three amino acids may be substituted for each other).

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for protease activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance,

crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

**Detergent compositions**

The detergent composition of the invention may be in any convenient form, e.g., a homogenous tablet, a tablet having two or more layers, a pouch having one or more compartments, a regular or compact powder, a granule, a paste, a gel, a bar, or a regular, compact or concentrated liquid.

In one embodiment, both the first protease and the second protease as described elsewhere herein are present in the same formulation, e.g. in the same liquid or solid phase. For example, in the case of a liquid formulation in the form of e.g. a liquid or gel, both the first and second protease would be present the liquid or gel phase. Similarly, in the case of a solid formulation, for example a powder, granulate or tablet, both the first and second protease would be present in the solid phase.

In a preferred embodiment, the detergent composition of the invention is a laundry composition, and in particular a liquid laundry composition. The use of two proteases with different net charge characteristics according to the invention allows flexibility with respect to incorporation into different liquid laundry detergent compositions, e.g. surfactant composition and/or level, and with respect to use under different wash conditions, e.g. wash pH and/or water hardness.

In one embodiment, the invention relates to a detergent composition as described above comprising at least a first protease and a second protease and further comprising one or more additional enzymes selected from the group consisting of amylases, catalases, cellulases (e.g., endoglucanases), cutinases, deoxyribonucleases, haloperoxygenases, lipases, mannanases, pectinases, pectin lyases, peroxidases, proteases, xanthanases, lichenases and xyloglucanases, or any mixture thereof.

The choice of additional components for a detergent composition is within the skill of the artisan and includes conventional ingredients, including the exemplary non-limiting components set forth below. The choice of components may include, for fabric care, the consideration of the type of fabric to be cleaned, the type and/or degree of soiling, the temperature at which cleaning is to take place, and the formulation of the detergent product.

In a particular embodiment, a detergent composition comprises the first and second protease and one or more non-naturally occurring detergent components, such as surfactants, hydrotropes, builders, co-builders, chelators or chelating agents, bleaching system or bleach components, polymers, fabric hueing agents, fabric conditioners, foam boosters, suds suppressors, dispersants, dye transfer inhibitors, fluorescent whitening agents, perfume, optical brighteners, bactericides, fungicides, soil suspending agents, soil release polymers, anti-redeposition agents, enzyme inhibitors or stabilizers, enzyme activators, antioxidants, and solubilizers. The detergent composition will typically comprise at least a surfactant and a builder.

In one embodiment, the protease may be added to a detergent composition in an amount corresponding to 0.01-200 mg of enzyme protein per liter of wash liquor, preferably 0.05-50 mg of enzyme protein per liter of wash liquor, in particular 0.1-10 mg of enzyme protein per liter of wash liquor.

A granulated composition for laundry may for example include 0.001%-20%, such as 0.01%-10%, such as 0.05%-5% of enzyme protein by weight of the composition.

An automatic dishwashing (ADW) composition may for example include 0.001%-30%, such as 0.01%-20%, such as 0.1-15%, such as 0.5-10% of enzyme protein by weight of the composition.

The enzymes such as the protease may be stabilized using conventional stabilizing agents, *e.g.,* a polyol such as propylene glycol or glycerol, a sugar or sugar alcohol, lactic acid, boric acid, or a boric acid derivative, *e.g.,* an aromatic borate ester, or a phenyl boronic acid derivative such as 4-formylphenyl boronic acid, and the composition may be formulated as described in, for example, WO 92/19709 and WO 92/19708 or the protease may be stabilized using peptide aldehydes or ketones such as described in WO 2005/105826 and WO 2009/118375.

The detergent composition may be formulated into a granular detergent for laundry. Such detergent may e.g. comprise;

a) at least 0.01 mg protease per gram of composition
b) anionic surfactant, preferably 5 wt % to 50 wt %
c) nonionic surfactant, preferably 1 wt % to 8 wt %
d) builder, preferably 5 wt % to 40 wt %, such as carbonates, zeolites, phosphate builder, calcium sequestering builders or complexing agents.

Although components mentioned below are categorized by general header according to a particular functionality, this is not to be construed as a limitation, as a component may comprise additional functionalities as will be appreciated by

the person skilled in the art.

Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized. Surfactants lower the surface tension in the detergent, which allows the stain being cleaned to be lifted and dispersed and then washed away.

When included therein, the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein, the detergent will usually contain from about 0% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein, the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl *N*-alkyl derivatives of glucosamine (glucamides, GA, or fatty acid glucamide, FAGA), as well as products available under the trade names SPAN and TWEEN, and combinations thereof.

When included therein, the detergent will usually contain from about 0% to about 10% by weight of a semipolar surfactant. Non-limiting examples of semipolar surfactants include amine oxides (AO) such as alkyldimethylamineoxide, *N*-(coco alkyl)-*N,N*-dimethylamine oxide and *N*-(tallow-alkyl)-*N,N*-bis(2-hydroxyethyl)amine oxide, fatty acid alkanolamides and ethoxylated fatty acid alkanolamides, and combinations thereof.

When included therein, the detergent will usually contain from about 0% to about 10% by weight of a zwitterionic surfactant. Non-limiting examples of zwitterionic surfactants include betaine, alkyldimethylbetaine, sulfobetaine, and combinations thereof.

Builders and Co-Builders

The detergent composition may contain about 0-65% by weight, such as about 5% to about 45% of a detergent builder or co-builder, or a mixture thereof. In a dishwashing detergent, the level of builder is typically 40-65%, particularly 50-65%. Builders and chelators soften, *e.g.,* the wash water by removing the metal ions form the liquid. The builder and/or co-builder may particularly be a chelating agent that forms water-soluble complexes with Ca and Mg. Any builder and/or co-builder known in the art for use in laundry detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (*e.g.,* SKS-6 from Hoechst), ethanolamines such as 2-aminoethan-1-ol (MEA), diethanolamine (DEA, also known as iminodiethanol), triethanolamine (TEA, also known as 2,2',2''-nitrilotriethanol), and carboxymethyl inulin (CMI), and combinations thereof.

In a preferred embodiment, the detergent composition is phosphate-free.

The detergent composition may also contain 0-20% by weight, such as about 5% to about 10%, of a detergent co-builder, or a mixture thereof. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N,N'*-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra-(methylenephosphonic acid) (EDTMPA), diethylenetriaminepentakis (methylenephosphonic acid) (DTPMPA or DTMPA), *N*-(2-hydroxyethyl)iminodiacetic acid (EDG), aspartic acid-*N*-monoacetic acid (ASMA), aspartic acid-*N,N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), N-methyliminodiacetic acid (MIDA), α-alanine-*N*, *N*-diacetic acid (α-ALDA), serine-*N*, *N*-diacetic acid (SEDA), isoserine-*N, N*-diacetic acid (ISDA), phenylalanine-*N*, *N*-diacetic acid (PHDA), anthranilic acid-*N, N*-diacetic acid (ANDA), sulfanilic acid-*N, N*-diacetic acid (SLDA), taurine-*N, N*-diacetic acid (TUDA) and sulfomethyl-*N*, *N*-diacetic acid (SMDA), *N*-(2-hydroxyethyl)-ethylidenediamine-*N*, *N', N'*-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, *e.g.,* WO 2009/102854 and US 5,977,053.

The first and second protease of the invention may also be formulated into a dishwashing composition, preferably an automatic dishwashing composition (ADW), comprising:

a) at least 0.01 mg of active protease, and
b) 10-50 wt % builder preferably selected from citric acid, methylglycine-N,N-diacetic acid (MGDA) and/or glutamic acid-N,N-diacetic acid (GLDA) and mixtures thereof, and
c) at least one bleach component.

<u>Bleaching Systems</u>

The detergent may contain 0-50% by weight, such as about 0.1% to about 25%, of a bleaching system. Bleach systems remove discolor often by oxidation, and many bleaches also have strong bactericidal properties, and are used for disinfecting and sterilizing. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator.

The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO 98/17767. A particular family of bleach activators of interest was disclosed in EP 624154 and particularly preferred in that family is acetyl triethyl citrate (ATC). ATC or a short chain triglyceride like triacetin has the advantage that it is environmentally friendly as it eventually degrades into citric acid and alcohol. Furthermore, acetyl triethyl citrate and triacetin have good hydrolytic stability in the product upon storage and are efficient bleach activators. Finally, ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst or a booster.

Some non-limiting examples of bleach catalysts that may be used in the compositions of the present invention include manganese oxalate, manganese acetate, manganese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2"-nitrilotris(ethane-1,2-diylazanylylidene-κN-methanylylidene)triphenolato-κ3O]manganese(III). The bleach catalysts may also be other metal

compounds, such as iron or cobalt complexes.

In some embodiments, the bleach component may be an organic catalyst selected from the group consisting of organic catalysts having the following formula:

(i)

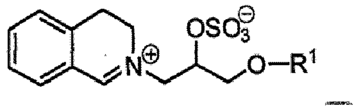

(ii)

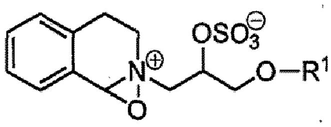

(iii) and mixtures thereof; wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, *e.g.,* in WO 2007/087258, WO 2007/087244, WO 2007/087259 and WO 2007/087242. Suitable photobleaches may for example be sulfonated zinc phthalocyanine.

Hydrotropes

A hydrotrope is a compound that solubilizes hydrophobic compounds in aqueous solutions (or oppositely, polar substances in a non-polar environment). Typically, hydrotropes have both hydrophilic and hydrophobic characters (so-called amphiphilic properties as known from surfactants); however, the molecular structures of hydrotropes generally do not favour spontaneous self-aggregation, see, *e.g.,* review by Hodgdon and Kaler, 2007, Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behaviour, stability, and colloidal properties of systems containing substances of polar and non-polar character, including mixtures of water, oil, surfactants, and polymers. Hydrotropes are classically used across industries from pharma, personal care and food to technical applications. Use of hydrotropes in detergent compositions allows for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers, hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole

(PVPVI). Further exemplary polymers include sulfonated polycarboxylates, polyethylene oxide and polypropylene oxide (PEO-PPO) and diquaternium ethoxy sulfate. Other exemplary polymers are disclosed in, *e.g.,* WO 2006/130575. Salts of the above-mentioned polymers are also contemplated.

Fabric hueing agents

The detergent compositions of the present invention may also include fabric hueing agents such as dyes or pigments, which when formulated in detergent compositions can deposit onto a fabric when the fabric is contacted with a wash liquor comprising the detergent compositions and thus altering the tint of the fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/003274, WO 2005/003275, WO 2005/003276 and EP 1876226 (hereby incorporated by reference). The detergent composition preferably comprises from about 0.00003 wt. % to about 0.2 wt. %, from about 0.00008 wt. % to about 0.05 wt. %, or even from about 0.0001 wt. % to about 0.04 wt. % fabric hueing agent. The composition may comprise from 0.0001 wt % to 0.2 wt. % fabric hueing agent, this may be especially preferred when the composition is in the form of a unit dose pouch. Suitable hueing agents are also disclosed in, *e.g.,* WO 2007/087257 and WO 2007/087243.

Additional Enzymes

A detergent additive or detergent composition may comprise one or more additional enzymes such as an amylase, an arabinase, a carbohydrase, a cellulase (*e.g.,* endoglucanase), a cutinase, a deoxyribonuclease, a galactanase, a haloperoxygenase, a lipase, a mannanase, an oxidase, *e.g.,* a laccase and/or peroxidase, a pectinase, a pectin lyase, an additional protease, a xylanase, a xanthanase or a xyloglucanase.

The properties of the selected enzyme(s) should be compatible with the selected detergent (*e.g.* pH-optimum, compatibility with other enzymatic and non-enzymatic ingredients, etc.).

Cellulases

Suitable cellulases include mono-component and mixtures of enzymes of bacterial or fungal origin. Chemically modified or protein engineered mutants are also contemplated. The cellulase may for example be a mono-component or a mixture of mono-component endo-1,4-beta-glucanase also referred to as endoglucanase.

Suitable cellulases include those from the genera *Bacillus, Pseudomonas, Humicola, Myceliophthora, Fusarium, Thielavia, Trichoderma,* and *Acremonium.* Exemplary cellulases include a fungal cellulase from *Humicola insolens* (US 4,435,307) or from *Trichoderma,* e.g. *T. reesei* or *T. viride.* Other suitable cellulases are from *Thielavia e.g. Thielavia terrestris* as described in WO 96/29397 or the fungal cellulases produced from *Myceliophthora thermophila* and *Fusarium oxysporum* disclosed in US 5,648,263, US 5,691,178, US 5,776,757, WO 89/09259 and WO 91/17244. Also relevant are cellulases from *Bacillus* as described in WO 02/099091 and JP 2000210081. Suitable cellulases are alkaline or neutral cellulases having care benefits. Examples of cellulases are described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Carezyme®, Carezyme® Premium, Celluzyme®, Celluclean®, Celluclast®, Endolase®, Renozyme®; Whitezyme® Celluclean® Classic, Cellusoft® (Novozymes A/S), Puradax®, Puradax HA, and Puradax EG (available from Genencor International Inc.) and KAC-500(B)™ (Kao Corporation).

Mannanases

Suitable mannanases include those of bacterial or fungal origin. Chemically or genetically modified mutants are included. The mannanase may be an alkaline mannanase of Family 5 or 26. It may be a wild-type from *Bacillus* or *Humicola,* particularly *B. agaradhaerens, B. licheniformis, B. halodurans, B. clausii,* or *H. insolens.* Suitable mannanases are described in WO 1999/064619. A commercially available mannanase is Mannaway® (Novozymes A/S).

Proteases

The composition may, in addition to the first and second proteases as disclosed herein, comprise one or more additional proteases including those of bacterial, fungal, plant, viral or animal origin. Proteases of microbial origin are preferred. The protease may be an alkaline protease, such as a serine protease or a metalloprotease. A serine protease may for example be of the S1 family, such as trypsin, or the S8 family such as subtilisin. A metalloprotease may for example be a thermolysin from, *e.g.,* family M4 or another metalloprotease such as those from M5, M7 or M8 families.

Examples of metalloproteases are the neutral metalloproteases as described in WO 2007/044993 (Genencor Int.) such as those derived from *Bacillus amyloliquefaciens.*

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Blaze®, Blaze Evity® 100T, Blaze Evity® 125T, Blaze Evity® 150T, Neutrase®, Everlase®, Esperase®, Progress® Uno and Progress® Excel (Novozymes A/S), those sold under the tradenames Maxatase®, Maxacal®, Maxapem®, Purafect®™, Purafect® Ox, Purafect® OxP, Purafect Prime®, Puramax®, FN2®, FN3®, FN4®, Excellase®, Excellenz P1000™, Excellenz P1250™, Eraser®, Preferenz® P100, Preferenz® P110, Effectenz P1000™, Effectenz P1050™, Effectenz P2000™, Purafast®, Properase®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

Lipases and Cutinases

Suitable lipases and cutinases include those of bacterial or fungal origin. Chemically modified or protein engineered mutant enzymes are included. Examples include lipase from *Thermomyces, e.g.,* from *T. lanuginosus* (previously named *Humicola lanuginosa*) as described in EP 258068 and EP 305216, cutinase from *Humicola, e.g., H. insolens* (WO 96/13580), lipase from strains of *Pseudomonas* (some of these now renamed to *Burkholderia*), *e.g., P. alcaligenes* or *P. pseudoalcaligenes* (EP 218272), *P. cepacia* (EP 331376), *P. sp.* strain SD705 (WO 95/06720 & WO 96/27002), *P. wisconsinensis* (WO 96/12012), GDSL-type *Streptomyces* lipases (WO 2010/065455), cutinase from *Magnaporthe grisea* (WO 2010/107560), cutinase from *Pseudomonas mendocina* (US 5,389,536), lipase from *Thermobifida fusca* (WO 2011/084412), *Geobacillus stearothermophilus* lipase (WO 2011/084417), lipase from *Bacillus subtilis* (WO 2011/084599), and lipase from *Streptomyces griseus* (WO 2011/150157) and *S. pristinaespiralis* (WO 2012/137147).

Other examples are lipase variants such as those described in EP 407225, WO 92/05249, WO 94/01541, WO 94/25578, WO 95/14783, WO 95/30744, WO 95/35381, WO 95/22615, WO 96/00292, WO 97/04079, WO 97/07202, WO 00/34450, WO 00/60063, WO 01/92502, WO 2007/87508 and WO 2009/109500.

Preferred commercial lipase products include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, *e.g.,* acyltransferases with homology to *Candida antarctica* lipase A (WO 2010/111143), acyltransferase from *Mycobacterium smegmatis* (WO 2005/056782), perhydrolases from the CE 7 family (WO 2009/067279), and variants of the *M. smegmatis* perhydrolase, in particular the S54V variant used in the commercial product Gentle Power Bleach from Huntsman Textile Effects Pte Ltd (WO 2010/100028).

Amylases

Suitable amylases which can be used together with the protease may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/19467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/10355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylases comprising residues 1-33 of the alpha-amylase derived from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred

variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from *B. amyloliquefaciens* shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T49I+G107A+H156Y+A181T+N190F+I201F+A209V+Q264S.

Other suitable amylases are amylases having the sequence of SEQ ID NO: 6 in WO 99/19467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/23873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/23873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 2008/153815, SEQ ID NO: 10 in WO 01/66712 or variants thereof having 90% sequence identity to SEQ ID NO: 2 of WO 2008/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 2009/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N 128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K,

wherein the variants are C-terminally truncated and optionally further comprise a substitution at position 243 and/or a deletion at position 180 and/or position 181.

Further suitable amylases are amylases having SEQ ID NO: 1 of WO 2013/184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or a deletion in position R178 and/or S179 or of T180 and/or G181. Most preferred amylase variants of SEQ ID NO: 1 comprise the substitutions:

E187P+I203Y+G476K
E187P+I203Y+R458N+T459S+D460T+G476K

and optionally further comprise a substitution at position 241 and/or a deletion at position 178 and/or position 179.

Further suitable amylases are amylases having SEQ ID NO: 1 of WO 2010/104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, I181, G182, M200, L204, E242, G477 and G478.

More preferred variants of SEQ ID NO: 1 are those having the substitution in one of more of the following positions: N21D, D97N, V128I K177L, M200L, L204YF, E242QA, G477K and G478K and/or a deletion in position R179 and/or S180 or of I181 and/or G182. Most preferred amylase variants of SEQ ID NO: 1 comprise the substitutions N210+D97N+V128I, and optionally further comprise a substitution at position 200 and/or a deletion at position 180 and/or position 181.

Other suitable amylases are the alpha-amylase having SEQ ID NO: 12 in WO 01/66712 or a variant having at least 90% sequence identity to SEQ ID NO: 12. Preferred amylase variants are those having a substitution, a deletion or an insertion in one of more of the following positions of SEQ ID NO: 12 in WO 01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particularly preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO 2011/098531, WO 2013/001078 and WO 2013/001087. Commercially available amylases include Duramyl™, Termamyl™, Fungamyl™, Stainzyme ™, Stainzyme Plus™, Natalase™, Liquozyme X, BAN™, Amplify® and Amplify® Prime (from Novozymes A/S), and Rapidase™, Purastar™/Effectenz™, Powerase, Preferenz S1000, Preferenz S100 and Preferenz S110 (from Genencor International Inc./DuPont).

One preferred amylase is a variant of the amylase having SEQ ID NO: 13 in WO 2016/180748 with the alterations H1*+N54S+ V56T+ K72R+G109A+ F113Q+ R116Q+ W167F+ Q172G+ A174S+ G182*+D183*+ G184T+ N195F+ V206L+ K391A+ P473R+ G476K.

Another preferred amylase is a variant of the amylase having SEQ ID NO: 1 in WO 2013/001078 with the alterations D183*+G184*+W140Y+N195F+V206Y+Y243F+E260G+ G304R+G476K.

Another preferred amylase is a variant of the amylase having SEQ ID NO: 1 in WO 2018/141707 with the alterations H1*+G7A+G109A+W140Y+G182*+D183*+N195F+V206Y+ Y243F+E260G+N280S+G304R+E391A+G476K.

A further preferred amylase is a variant of the amylase having SEQ ID NO: 1 in WO 2017/191160 with the alterations L202M + T246V.

Peroxidases/Oxidases

Suitable peroxidases/oxidases include those of plant, bacterial or fungal origin. Chemically modified or protein engineered mutants are included. Examples of useful peroxidases include peroxidases from *Coprinus, e.g.,* from *C. cinereus,* and variants thereof as those described in WO 93/24618, WO 95/10602, and WO 98/15257.

Commercially available peroxidases include Guardzyme™ (Novozymes A/S).

Deoxyribonucleases (DNases)

Suitable deoxyribonucleases (DNases) are any enzyme that catalyzes the hydrolytic cleavage of phosphodiester linkages in the DNA backbone, thus degrading DNA. Bacterial DNases are preferred, in particular a DNase which is obtainable from a species of *Bacillus* is preferred, in particular a DNase which is obtainable from *Bacillus subtilis* or *Bacillus licheniformis.* Examples of such DNases are described in WO 2011/098579 and WO 2014/087011.

Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants: The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant Science Series, volume 71, Marcel Dekker, Inc., 1997.

Dye Transfer inhibiting Agents: The detergent compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001% to about 10%, from about 0.01% to about 5% or even from about 0.1% to about 3% by weight of the composition.

Fluorescent whitening agent: The detergent compositions of the present invention will preferably also contain additional components that may tint articles being cleaned, such as fluorescent whitening agent or optical brighteners. Where present the brightener is preferably at a level of about 0.01% to about 05%. Any fluorescent whitening agent suitable for use in a laundry detergent composition may be used in the composition of the present invention. The most commonly used fluorescent whitening agents are those belonging to the classes of diaminostilbene-sulphonic acid derivatives, diarylpyrazoline derivatives and bisphenyl-distyryl derivatives. Examples of the diaminostilbene-sulphonic acid derivative type of fluorescent whitening agents include the sodium salts of: 4,4'-bis-(2-diethanolamino-4-anilino-s-triazin-6-ylamino) stilbene-2,2'-disulphonate; 4,4'-bis-(2,4-dianilino-s-triazin-6-ylamino) stilbene-2.2'-disulphonate; 4,4'-bis-(2-anilino-4(N-methyl-N-2-hydroxy-ethylamino)-s-triazin-6-ylamino) stilbene-2,2'-disulphonate, 4,4'-bis-(4-phenyl-2,1,3-triazol-2-yl)stilbene-2,2'-disulphonate; 4,4'-bis-(2-anilino-4(1-methyl-2-hydroxy-ethylamino)-s-triazin-6-ylamino) stilbene-2,2'-disulphonate and 2-(stilbyl-4"-naptho-1.,2':4,5)-1,2,3-trizole-2"-sulphonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4 anilino-s-triazin-6-ylamino) stilbene disulphonate. Tinopal CBS is the disodium salt of 2,2'-bis-(phenyl-styryl) disulphonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins. Suitable fluorescent brightener levels include lower levels of from about 0.01, from 0.05, from about 0.1 or even from about 0.2 wt. % to upper levels of 0.5 or even 0.75 wt. %.

Soil release polymers: The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore, random graft co-polymers are suitable soil release polymers Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose deriviatives such as those described in EP 1867808 or WO 03/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents: The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

**Formulation of Detergent Products**

The detergent enzymes, i.e. the first and second proteases and optionally one or more additional enzymes, may be included in a detergent composition by adding separate additives containing one or more enzymes, or by adding a combined additive comprising these enzymes.

As noted above, the detergent composition may, *e.g.,* be in the form of a powder, a granulate, a tablet, a pouch, a paste, a gel or a liquid.

Pouches

Pouches (pods) can be configured as single or multiple compartments and can be of any form, shape and material suitable to hold the composition, without allowing the release of the composition from the pouch prior to water contact. The pouch is made from water soluble film which encloses an inner volume. The inner volume can be divided into compartments of the pouch. Preferred films are polymeric materials, preferably polymers which are formed into a film or sheet. Preferred polymers, copolymers or derivates thereof are selected from polyacrylates, and water-soluble acrylate copolymers, methyl cellulose, carboxy methyl cellulose, sodium dextrin, ethyl cellulose, hydroxyethyl cellulose, hydroxypropyl methyl cellulose, maltodextrin, polymethacrylates, most preferably polyvinyl alcohol copolymers and hydroxypropyl methyl cellulose (HPMC). Preferably the level of polymer in the film for example PVA is at least about 60%. The preferred average molecular weight will typically be about 20,000 to about 150,000. Films can also be of blend compositions comprising hydrolytically degradable and water-soluble polymer blends such as polylactide and polyvinyl alcohol (known under the Trade reference M8630 as sold by Chris Craft In. Prod. of Gary, Indiana, US) plus plasticizers like glycerol, ethylene glycerol, propylene glycol, sorbitol and mixtures thereof. The pouches can for example comprise a solid laundry detergent composition or part components and/or a liquid cleaning composition or part components separated by the water-soluble film. The compartment for liquid components can be different in composition than compartments containing solids. *See, e.g.,* US 2009/0011970.

Detergent ingredients can be separated physically from each other by compartments in water dissolvable pouches or in different layers of tablet, thereby avoiding negative storage interaction between components. Different dissolution profiles of each of the compartments can also give rise to delayed dissolution of selected components in the wash solution.

Liquids and gels

A liquid or gel detergent which is not unit dosed may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may also be non-aqueous.

Granular detergent formulations

Enzymes in the form of granules, comprising an enzyme-containing core and optionally one or more coatings, are commonly used in granular (powder) detergents. Various methods for preparing the core are well-known in the art and include, for example, a) spray drying of a liquid enzyme-containing solution, b) production of layered products with an enzyme coated as a layer around a pre-formed inert core particle, e.g. using a fluid bed apparatus, c) absorbing an enzyme onto and/or into the surface of a pre-formed core, d) extrusion of an enzyme-containing paste, e) suspending an enzyme-containing powder in molten wax and atomization to result in prilled products, f) mixer granulation by adding an enzyme-containing liquid to a dry powder composition of granulation components, g) size reduction of enzyme-containing cores by milling or crushing of larger particles, pellets, etc., and h) fluid bed granulation. The enzyme-containing cores may be dried, e.g. using a fluid bed drier or other known methods for drying granules in the feed or enzyme industry, to result in a water content of typically 0.1 -10% w/w water.

The enzyme-containing cores are optionally provided with a coating to improve storage stability and/or to reduce dust formation. One type of coating that is often used for enzyme granulates for detergents is a salt coating, typically an inorganic salt coating, which may e.g. be applied as a solution of the salt using a fluid bed. Other coating materials that may be used are, for example, polyethylene glycol (PEG), methyl hydroxy-propyl cellulose (MHPC) and polyvinyl alcohol (PVA). The granules may contain more than one coating, for example a salt coating followed by an additional coating of a material such as PEG, MHPC or PVA.

For further information on enzyme granules and production thereof, see WO 2013/007594 as well as e.g. WO 2009/092699, EP 1705241, EP 1382668, WO 2007/001262, US 6,472,364, WO 2004/074419 and WO 2009/102854.

Formulation of enzyme in co-granule

The enzyme of the invention may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulates for the detergent industry are disclosed in the IP.com disclosure IPCOM000200739D.

Another example of formulation of enzymes by the use of co-granulates are disclosed in WO 2013/188331, which relates to a detergent composition comprising (a) a multi-enzyme co-granule; (b) less than 10 wt% zeolite (anhydrous basis); and (c) less than 10 wt% phosphate salt (anhydrous basis), wherein said enzyme co-granule comprises from 10 to 98 wt% moisture sink components and the composition additionally comprises from 20 to 80 wt% detergent moisture sink components.

WO 2013/188331 also relates to a method of treating and/or cleaning a surface, preferably a fabric surface comprising the steps of (i) contacting said surface with the detergent composition as claimed and described herein in an aqueous wash liquor, (ii) rinsing and/or drying the surface.

The multi-enzyme co-granule may comprise an enzyme of the invention and (a) one or more enzymes selected from the group consisting of first- wash lipases, cleaning cellulases, xyloglucanases, perhydrolases, peroxidases, lipoxygenases, laccases and mixtures thereof; and (b) one or more enzymes selected from the group consisting of hemicellulases, proteases, care cellulases, cellobiose dehydrogenases, xylanases, phospho lipases, esterases, cutinases, pectinases, mannanases, pectate lyases, keratinases, reductases, oxidases, phenoloxidases, ligninases, pullulanases, tannases, pentosanases, lichenases glucanases, arabinosidases, hyaluronidase, chondroitinase, amylases, and mixtures thereof.

Laundry soap bars

The polypeptides of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

Bar soaps for hand laundry may be in the form of either "soap bars" (oil-based) or "non-soap detergent" (NSD) bars. As the name implies, non-soap detergent bars are characterized by a lack of fatty acid soap ingredients from plant or animal sources, and instead are based on synthetic detergents such as LAS (linear alkylbenzene sulfonate).

Laundry bars comprising the first and second protease of the invention may be produced by methods conventionally known and used to produce soap bars and using conventional laundry bar making equipment such as but not limited to: mixers, plodders, e.g. a two-stage vacuum plodder, extruders, roll mills, cutters, logo-stampers, cooling tunnels and wrappers.

**Uses**

The present invention is also directed to methods for using the detergent compositions in laundering of textiles and fabrics, including household laundry and industrial laundry applications. The invention also relates to use of a composition of the present in a cleaning process, such as laundry or hard surface cleaning such as dishwashing. In preferred embodiment, the compositions of the invention are designed for and used in laundry applications. In a further preferred embodiment, the composition may be in the form of a liquid laundry formulation.

A detergent composition of the present invention may be formulated, for example, as a hand or machine laundry detergent composition including a laundry additive composition suitable for pretreatment of stained fabrics and a rinse added fabric softener composition, or be formulated as a detergent composition for use in general household hard surface cleaning operations, or be formulated for hand or machine dishwashing operations.

The cleaning process or the textile care process may for example be a laundry process, a dishwashing process or cleaning of hard surfaces such as bathroom tiles, floors, table tops, drains, sinks and washbasins. Laundry processes can for example be household laundering, but may also be industrial laundering. Furthermore, the invention relates to a process for laundering of fabrics and/or garments, where the process comprises treating fabrics with a washing solution containing a detergent composition of the invention. The cleaning process or a textile care process can for example be carried out in a machine washing or manually. The washing solution can for example be an aqueous washing solution containing a detergent composition.

The invention further concerns the use of the detergent compositions in a proteinaceous stain removing process. The proteinaceous stains may be stains such as food stains, e.g., baby food, cocoa, egg or milk, or other stains such as sebum, blood, ink or grass, or a combination hereof.

This aspect further relates to a method of cleaning, especially for cleaning fabrics or textiles, or for dishwashing, comprising contacting fabrics/textiles or dishes with the detergent composition of this aspect under conditions suitable for cleaning the fabrics/textiles or dishes.

The first and second proteases in the composition according to this aspect, and for use thereof and a method of

cleaning, may be any of the proteases described herein.

**Washing Method**

The present invention provides a method of cleaning, especially for cleaning fabrics or textiles, i.e. laundry, or for dishwashing, with a detergent composition of the invention comprising a first protease and a second protease.

The method of cleaning comprises contacting an object with a detergent composition comprising the first and second protease under conditions suitable for cleaning the object. In a preferred embodiment the detergent composition is used in a laundry process.

Another embodiment relates to a method for removing stains from fabrics or textiles, which comprises contacting the fabric or textile with a composition of the invention under conditions suitable for cleaning the object.

Another embodiment relates to a method for removing stains from dishware, which comprises contacting the dishware with a composition of the invention under conditions suitable for cleaning the object.

The compositions may be employed at concentrations from about 100 ppm, preferably 500 ppm to about 15,000 ppm in solution. The water temperatures typically range from about 5°C to about 95°C, including about 10°C, about 15°C, about 20°C, about 25°C, about 30°C, about 35°C, about 40°C, about 45°C, about 50°C, about 55°C, about 60°C, about 65°C, about 70°C, about 75°C, about 80°C, about 85°C and about 90°C. The water to fabric ratio is typically from about 1:1 to about 30:1.

The enzymes of the detergent composition of the invention may be stabilized using conventional stabilizing agents and protease inhibitors, *e.g.,* a polyol such as propylene glycol or glycerol, a sugar or sugar alcohol, different salts such as NaCl; KCl; lactic acid, formic acid, boric acid, or a boric acid derivative, *e.g.,* an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO 2009/118375, WO 98/13459) or a protease inhibitor of the protein type such as RASI, BASI, WASI (bifunctional alpha-amylase/subtilisin inhibitors of rice, barley and wheat) or CI2 or SSI. The composition may be formulated as described in, *e.g.,* WO 92/19709, WO 92/19708 and US 6,472,364. In some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), Tin (II), cobalt (II), copper (II), Nickel (II), and oxovanadium (IV)).

**Methods of production**

The first and second protease may be produced by standard methods that are well-known in the art, using cultivation of host cells cultivated in a suitable nutrient medium

The host cell may be any cell useful in recombinant enzyme production, *e.g.,* a prokaryote or a eukaryote.

The prokaryotic host cell will typically be a Gram-positive or Gram-negative bacterium, such as a Gram-positive bacterium selected from *Bacillus, Clostridium, Enterococcus, Geobacillus, Lactobacillus, Lactococcus, Oceanobacillus, Staphylococcus, Streptococcus* and *Streptomyces,* or a Gram-negative bacterium selected from *Campylobacter, E. coli, Flavobacterium, Fusobacterium, Helicobacter, Ilyobacter, Neisseria, Pseudomonas, Salmonella* and *Ureaplasma.*

The bacterial host cell may e.g. be a *Bacillus* cell selected from *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis* and *Bacillus thuringiensis* cells.

For information on suitable host cells, see e.g. WO 2017/207762.

Host cells may, for example, be cultivated by shake flask cultivation, or small-scale or large-scale fermentation (including continuous, batch, fed-batch, or solid-state fermentations) in laboratory or industrial fermentors performed in a suitable medium and under conditions allowing the variant to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the variant is secreted into the nutrient medium, the variant can be recovered directly from the medium. If the variant is not secreted, it can be recovered from cell lysates.

The variant may be detected using methods known in the art that are specific for the variants with protease activity, and may be recovered and purified using methods known in the art. See e.g. WO 2017/207762 for further information.

The present invention is further described by the following examples that should not be construed as limiting the scope of the invention.

## EXAMPLES

### Materials and methods

Preparation and purification of polypeptides

Mutation and introduction of expression cassettes into *Bacillus subtilis* was performed by standard methods known in the art. All DNA manipulations were performed by PCR (e.g. as described by Sambrook et al., Molecular Cloning; 3rd Ed., 2001, Cold Spring Harbor Laboratory Press) using standard methods known to the skilled person.

Recombinant *B. subtilis* constructs encoding subtilase polypeptides were inoculated into and cultivated in a complex medium (TBgly) for 24h at 37°C. Shake flasks containing a rich media (PS-1: 100 g/L Sucrose (Danisco cat.no. 109-0429), 40 g/L crust soy (soy bean flour), 10g/L $Na_2HPO_4.12H_2O$ (Merck cat.no. 106579), 0.1ml/L Dowfax63N10 (Dow) were inoculated in a ratio of 1:100 with the overnight culture. Shake flask cultivation was performed for 4 days at 30°C shaking at 270 rpm.

Purification of culture supernatants was performed as follows:

The culture broth is centrifuged at 26000 x g for 20 minutes and the supernatant is carefully decanted from the precipitate. The supernatant is filtered through a Nalgene 0.2 μm filtration unit in order to remove the remains of the host cells. The pH in the 0.2 μm filtrate is adjusted to pH 8 with 3 M Tris base and the pH-adjusted filtrate is applied to a MEP Hypercel column (Pall Corporation) equilibrated in 20 mM Tris/HCl, 1 mM $CaCl_2$, pH 8.0. After washing the column with the equilibration buffer, the column is step-eluted with 20 mM $CH_3COOH$/NaOH, 1 mM $CaCl_2$, pH 4.5. Fractions from the column are analyzed for protease activity using the Suc-AAPF-pNA assay at pH 9 and peak fractions are pooled. The pH of the pool from the MEP Hypercel column is adjusted to pH 6 with 20% (v/v) $CH_3COOH$ or 3 M Tris base and the pH-adjusted pool is diluted with deionized water to the same conductivity as 20 mM MES/NaOH, 2 mM $CaCl_2$, pH 6.0. The diluted pool is applied to an SP-Sepharose® Fast Flow column (GE Healthcare) equilibrated in 20 mM MES/NaOH, 2 mM $CaCl_2$, pH 6.0. After washing the column with the equilibration buffer, the protease variant is eluted with a linear NaCl gradient (0 → 0.5 M) in the same buffer over five column volumes. Fractions from the column are analyzed for protease activity using the Suc-AAPF- pNA assay at pH 9 and active fractions are analyzed by SDS-PAGE. Fractions in which only one band is observed on the Coomassie stained SDS-PAGE gel are pooled as the purified preparation and used for further experiments.

Automatic Mechanical Stress Assay (AMSA) for laundry

Experiments were performed to assess the wash performance of selected protease variants in laundry detergent compositions. The proteases were tested using the Automatic Mechanical Stress Assay (AMSA). With the AMSA, the wash performance of a large quantity of small volume enzyme-detergent solutions can be examined. The AMSA plate has a number of slots for test solutions and a lid firmly squeezing the test sample to be washed (a textile swatch for testing a laundry detergent) against the slot openings. During the washing time, the plate, test solutions, test sample and lid are vigorously shaken to bring the test solution into contact with the soiled test sample and to apply mechanical stress in a regular, periodic oscillating manner. For further description see WO 02/42740 especially the paragraph "Special method embodiments" at page 23-24.

The performance of the enzyme variants and blends thereof was in this example measured as the brightness of the colour of textile samples washed with a specific protease or a protease blend. The brightness can be expressed as the intensity of the light reflected from the textile sample when illuminated with white light. When the textile is stained, the intensity of the reflected light is lower than that of a clean textile. Therefore, the intensity of the reflected light can be used to measure wash performance of the proteases and the protease blends.

Colour measurements are made with a professional flatbed scanner (Epson Expression 10000XL), which is used to capture an image of the washed textile samples.

To extract a value for the light intensity from the scanned images, a specially designed software application is used (*Novozymes Color Vector Analyzer*). The program retrieves the values from the image and converts them into values for red, green and blue (RGB). The intensity value (Int) is calculated by adding the RGB values together as vectors and then taking the length of the resulting vector:

$$Int = \sqrt{r^2 + g^2 + b^2}$$

Standard textile pieces were obtained from Center for Testmaterials BV, P.O. Box 120, 3133 KT Vlaardingen, The Netherlands. The detergent for the wash performance tests was a liquid laundry model detergent. The composition of the detergent as well as other test parameters are given in the table below.

Table 1: Detergent composition and test conditions for AMSA

| | |
|---|---|
| Liquid laundry model detergent | Sodium lauryl ether sulfate 28%) 17.63%<br>(C10-C13) Alkylbenzene-sulfonic acid (48%) 12%<br>Alcohol ethoxylate with 8 mol EO (ca. 100%) 11%<br>Propane-1,2-diol (>98%) 6%<br>Triethanolamine (100%) 3.33%<br>9/1 Ethanol:propan-2-ol (90/10%) 3%<br>Soy fatty acid (>90%) 2.75%<br>Coco fatty acid (>99%) 2.75%<br>Sodium citrate (100%) 2%<br>Sodium hydroxide (>99%) 1.75%<br>Sodium formate (>95%) 1%<br>Diethylenetriaminepentakis(methylene)pentakis(phosphonic acid), heptasodium salt (DTMPA-Na7) (about 42%) 0,48%<br>Coploy(acrylic acid/maleic acid), sodium salt (about 40%) 0.46%<br>Water 34.14% |
| Detergent dosage | 3.33 g/L |
| Test solution volume | 160 micro L |
| pH | As is (7.6-7.7) |
| Wash time | 20 minutes |
| Temperature | 20°C |
| Water hardness | 15°dH |
| Enzyme concentrations in test solution | 0.25-0.5-1-2 mg enzyme protein/liter, either as single protease, or as total concentration in the 1:1-blend of two proteases, respectively. |
| Test materials | PC-03 (polyester/cotton textile stained with chocolate milk with carbon black), and PC-10 (polyester/cotton textile stained with pigment, oil and milk), obtained from Center for Testmaterials BV, P.O. Box 120, 3133 KT Vlaardingen, The Netherlands. |

Table 2 outlines the proteases that were tested. These were SEQ ID NO: 1 and variants thereof, with position numbers based on the numbering of SEQ ID NO: 2, and with the net formal charge relative to SEQ ID NO: 1 indicated.

Table 2: Proteases tested in charge combinations

| Variant | Net charge |
|---|---|
| S99D+S101E+S103A+V104I+S156D+G160S+L262E | -4 |
| K27H+S99D+S101E+S103A+V104I+S156D+G160S+Q245R+L262E | -4 |
| S9R+S99D+S101E+S103A+V104I+S156D+G160S+K235M+Q245R+L262E | -3 |
| S9R+S99D+S101E+S103A+V104I+S156D+G160S+L262E | -3 |
| S9R+K27M+S99D+S101E+S103A+V104I+S156D+G160S+K237M+Q245R+L262E | -4 |
| S9E+N43R+N76D+V205I+Q206L+Y209W+S259D+N261W+L262E | -3 |
| S9E+N43R+N76D+N185E+S188E+Q191N+A194P+Q206L+Y209W+S259D+L262E | -5 |
| *36D+N76D+H120D+G195E+K235L | -5 |
| K27M | -1 |
| SEQ ID NO: 1 | 0 |
| S99AD | -1 |
| G97D+Y209W+A215K | 0 |
| G97D+N117R+Y209W+A215K | +1 |

(continued)

| Variant | Net charge |
|---|---|
| G97D+S156D+Y209W+A215K | -1 |
| G97D+Y209W+A215K+L262E | -1 |
| N62D+N76D+G97D+Y209W+A215K+L262E | -3 |
| N62D+G97D+S101E+V177I+Y209W+A215K+L262E | -3 |
| N62D+G97D+S101E+Y209W+A215K+L262E | -3 |
| G97D+S101E+S156D+A172V+Y209W+A215K+L262E | -3 |
| N76D+G97D+N140D+S156D+Y209W+A215K+L262E | -4 |
| K27M+N77D+G97D+S156D+Y209W+A215K+L262E | -4 |
| G97D+S156D+Y209W+A215K+L262E | -2 |
| S9R+S99D+S101E+S103A+V104I+S156D+G160S+Q245R+L262E | -2 |

**Example 1**

Wash performance of different variants of SEQ ID NO: 1 with the mutations indicated in Tables 3 and 4 below having a net charge of -4 or -3 relative to SEQ ID NO: 1 were tested in AMSA as described above either alone ("single enzyme") or together with a variant of SEQ ID NO: 1 having the substitution K27M and a net charge of -1 relative to SEQ ID NO: 1. The wash performance of the K27M variant alone was also tested in AMSA.

For each enzyme concentration (0.25, 0.5, 1 and 2 mg enzyme protein/liter as described above) a delta intensity value was calculated for the single enzymes and the 1:1 mixtures as the intensity value (Int) of a test material washed with the detergent containing a single enzyme or mixture minus the intensity value of a test material washed with the detergent alone, i.e. without any enzyme. The delta intensity values for each of the four enzyme concentrations were added together for each treatment (single enzyme or 1:1 mixture) to obtain a sum of delta intensity for each treatment for each of the two stains PC-10 and PC-03.

The sums of delta intensity for the 1:1 mixtures were then compared to the sums of delta intensity for the single enzymes in the mixture, and the relative performance of the mixture compared to a single enzyme was determined for the PC-10 and PC-03 stains. The relative performance values in Tables 3 and 4 below for the individual stains PC-10 and PC-03, expressed as percent relative performance against a single enzyme, were determined by dividing the sum of delta intensity for a mixture by the sum of delta intensity for a single enzyme.

In Table 3 the mixtures are compared to single enzymes with a net formal charge of -4 or -3 relative to SEQ ID NO: 1 (enzymes (a), (b), (c), (d) in the column "Variant / single enzyme"), while Table 4 compares the mixtures to the K27M variant alone.

Finally, to give an indication of the overall performance of the mixtures compared to the single enzymes on a set of different protein-based stains, the last column in each table, "PC-03 + PC-10", provides the percent relative performance of the mixtures compared to single enzymes calculated in the same manner as described above for the individual stains PC-10 and PC-03. In this case, however, the sums of delta intensity for a mixture on both PC-10 and PC-03 were added together and divided by the sums of delta intensity for a single enzyme on both PC-10 and PC-03.

Table 3: Relative performance of charge mixtures with variant K27M against single enzymes (a), (b), (c), (d)

| | | Relative performance against single enzyme | | |
|---|---|---|---|---|
| Variant / single enzyme | Δ Net charge (rel. to SEQ ID NO: 1) | PC-10 | PC-03 | PC-03 + PC-10 |
| (a) S99D S101E S103A V104I S156D G160S L262E | -4 | 299% | 134% | 165% |
| (b) K27H S99D S101E S103A V104I S156D G160S Q245R L262E | -4 | 179% | 122% | 138% |
| (c) S9R S99D S101E S103A V104I S156D G160S K235M Q245R L262E | -3 | 122% | 106% | 112% |

(continued)

| | | Relative performance against single enzyme | | |
|---|---|---|---|---|
| Variant / single enzyme | Δ Net charge (rel. to SEQ ID NO: 1) | PC-10 | PC-03 | PC-03 + PC-10 |
| (d) S9R K27M S99D S101E S103A V104I S156D G160S K237M Q245R L262E | -4 | 107% | 103% | 105% |

Table 4: Relative performance of charge mixtures with variant K27M against variant K27M alone

| | | Relative performance against variant K27M | | |
|---|---|---|---|---|
| Variant | Δ Net charge (rel. to SEQ ID NO: 1) | PC-10 | PC-03 | PC-03 + PC-10 |
| S99D S101E S103A V104I S156D G160S L262E | -4 | 105% | 110% | 108% |
| K27H S99D S101E S103A V104I S156D G160S Q245R L262E | -4 | 116% | 114% | 114% |
| S9R S99D S101E S103A V104I S156D G160S K235M Q245R L262E | -3 | 156% | 118% | 131% |
| S9R K27M S99D S101E S103A V104I S156D G160S K237M Q245R L262E | -4 | 137% | 114% | 122% |

**Example 2**

Wash performance of different variants of SEQ ID NO: 1 with the mutations indicated in Tables 5 and 6 below having a net charge of -4 relative to SEQ ID NO: 1 were tested in AMSA as described above either alone ("single enzyme") or together with SEQ ID NO: 1 (Savinase®). The wash performance of SEQ ID NO: 1 alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 5 the mixtures are compared to single enzymes with a net formal charge of -4 relative to SEQ ID NO: 1 (enzymes (a), (b), (c) in the column "Variant / single enzyme"), while Table 6 compares the mixtures to SEQ ID NO: 1 alone.

Table 5: Relative performance of charge mixtures with SEQ ID NO: 1 against single enzymes (a), (b), (c)

| | | Relative performance against single enzyme | | |
|---|---|---|---|---|
| Variant / single enzyme | Δ Net charge (rel. to SEQ ID NO: 1) | PC-10 | PC-03 | PC-03 + PC-10 |
| (a) S99D S101E S103A V104I S156D G160S L262E | -4 | 188% | 121% | 138% |
| (b) K27H S99D S101E S103A V104I S156D G160S Q245R L262E | -4 | 144% | 112% | 120% |
| (c) S9R K27M S99D S101E S103A V104I S156D G160S K237M Q245R L262E | -4 | 102% | 100% | 101% |

Table 6: Relative performance of charge mixtures with SEQ ID NO: 1 against SEQ ID NO: 1 alone

| | | Relative performance against SEQ ID NO: 1 | | |
|---|---|---|---|---|
| Variant | Δ Net charge (rel. to SEQ ID NO: 1) | PC-10 | PC-03 | PC-03 + PC-10 |
| S99D S101E S103A V104I S156D G160S L262E | -4 | 108% | 105% | 106% |
| K27H S99D S101E S103A V104I S156D G160S Q245R L262E | -4 | 118% | 125% | 123% |
| S9R K27M S99D S101E S103A V104I S156D G160S K237M Q245R L262E | -4 | 150% | 132% | 138% |

**Example 3**

Wash performance of different variants of SEQ ID NO: 1 with the mutations indicated in Tables 7 and 8 below having a net charge of -4 or -3 relative to SEQ ID NO: 1 were tested in AMSA as described above either alone ("single enzyme") or together with a variant of SEQ ID NO: 1 having the mutation S99AD (net charge of -1 relative to SEQ ID NO: 1). The wash performance of the S99AD variant alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 7 the mixtures are compared to single enzymes with a net formal charge of 4 or 3 relative to SEQ ID NO: 1 (enzymes (a), (b), (c), (d), (e) in the column "Variant / single enzyme"), while Table 8 compares the mixtures to the S99AD variant alone.

Table 7: Relative performance of charge mixtures with variant S99AD against single enzymes (a), (b), (c), (d), (e)

| | | Relative performance against single enzyme | | |
|---|---|---|---|---|
| Variant / single enzyme | Δ Net charge (rel. to SEQ ID NO: 1) | PC-10 | PC-03 | PC-03 + PC-10 |
| (a) S99D S101E S103A V104I S156D G160S L262E | -4 | 527% | 109% | 180% |
| (b) K27H S99D S101E S103A V104I S156D G160S Q245R L262E | -4 | 144% | 106% | 122% |
| (c) S9R S99D S101E S103A V104I S156D G160S K235M Q245R L262E | -3 | 120% | 91% | 103% |
| (d)S9R S99D S101E S103A V104I S156D G160S L262E | -3 | 144% | 82% | 102% |
| (e) S9R K27M S99D S101E S103A V104I S156D G160S K237M Q245R L262E | -4 | 151% | 87% | 106% |

Table 8: Relative performance of charge mixtures with variant S99AD against variant S99AD alone

| | | Relative performance against variant S99AD | | |
|---|---|---|---|---|
| Variant | Δ Net charge (rel. to SEQ ID NO: 1) | PC-10 | PC-03 | PC-03 + PC-10 |
| S99D S101E S103A V104I S156D G160S L262E | -4 | 122% | 113% | 117% |
| K27H S99D S101E S103A V104I S156D G160S Q245R L262E | -4 | 127% | 117% | 121% |

(continued)

| | | Relative performance against variant S99AD | | |
|---|---|---|---|---|
| Variant | Δ Net charge (rel. to SEQ ID NO: 1) | PC-10 | PC-03 | PC-03 + PC-10 |
| S9R S99D S101E S103A V104I S156D G160S K235M Q245R L262E | -3 | 132% | 119% | 126% |
| S9R S99D S101E S103A V104I S156D G160S L262E | -3 | 121% | 137% | 129% |
| S9R K27M S99D S101E S103A V104I S156D G160S K237M Q245R L262E | -4 | 110% | 136% | 124% |

**Example 4**

Wash performance of different variants of SEQ ID NO: 1 with the mutations indicated in Tables 9 and 10 below having a net charge of -5, -4 or -3 relative to SEQ ID NO: 1 were tested in AMSA as described above either alone ("single enzyme") or together with a variant of SEQ ID NO: 1 having the substitutions G97D Y209W A215K (net charge of 0 relative to SEQ ID NO: 1). The wash performance of the G97D Y209W A215K variant alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 9 the mixtures are compared to single enzymes with a net formal charge of -5, -4 or -3 relative to SEQ ID NO: 1 (enzymes (a), (b), (c), (d) in the column "Variant / single enzyme"), while Table 10 compares the mixtures to the G97D Y209W A215K variant alone.

Table 9: Relative performance of charge mixtures with variant G97D Y209W A215K against single enzymes (a), (b), (c), (d)

| | | Relative performance against single enzyme | | |
|---|---|---|---|---|
| Variant / single enzyme | Δ Net charge (rel. to SEQ ID NO: 1) | PC-10 | PC-03 | PC-03 + PC-10 |
| (a) S99D S101E S103A V104I S156D G160S L262E | -4 | 191% | 118% | 139% |
| (b) S9E N43R N76D V205I Q206L Y209W S259D N261W L262E | -3 | 132% | 116% | 123% |
| (c) S9E N43R N76D N185E S188E Q191N A194P Q206L Y209W S259D L262E | -5 | 164% | 126% | 141% |
| (d) *36D N76D H120D G195E K235L | -5 | 116% | 102% | 107% |

Table 10: Relative performance of charge mixtures with variant G97D Y209W A215K against variant G97D Y209W A215K alone

| | | Relative performance against variant G97D Y209W A215K | | |
|---|---|---|---|---|
| Variant | Δ Net charge (rel. to SEQ ID NO: 1) | PC-10 | PC-03 | PC-03 + PC-10 |
| S99D S101E S103A V104I S156D G160S L262E | -4 | 109% | 141% | 126% |
| S9E N43R N76D V205I Q206L Y209W S259D N261W L262E | -3 | 111% | 114% | 113% |

(continued)

| | | Relative performance against variant G97D Y209W A215K | | |
|---|---|---|---|---|
| Variant | Δ Net charge (rel. to SEQ ID NO: 1) | PC-10 | PC-03 | PC-03 + PC-10 |
| S9E N43R N76D N185E S188E Q191N A194P Q206L Y209W S259D L262E | -5 | 112% | 115% | 114% |
| *36D N76D H120D G195E K235L | -5 | 115% | 125% | 120% |

**Example 5**

Wash performance of different variants of SEQ ID NO: 1 with the mutations indicated in Tables 11 and 12 below having a net charge of -5, -4 or -3 relative to SEQ ID NO: 1 were tested in AMSA as described above either alone ("single enzyme") or together with a variant of SEQ ID NO: 1 having the substitutions G97D N117R Y209WA215K (net charge of +1 relative to SEQ ID NO: 1). The wash performance of the G97D N117R Y209W A215K variant alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 11 the mixtures are compared to single enzymes with a net formal charge of -5, -4 or -3 relative to SEQ ID NO: 1 (enzymes (a), (b), (c) in the column "Variant / single enzyme"), while Table 12 compares the mixtures to the G97D N117R Y209W A215K variant alone.

Table 11: Relative performance of charge mixtures with variant G97D N117R Y209W A215K against single enzymes (a), (b), (c)

| | | Relative performance against single enzyme | | |
|---|---|---|---|---|
| Variant / single enzyme | Δ Net charge (rel. to SEQ ID NO: 1) | PC-10 | PC-03 | PC-03 + PC-10 |
| (a) S99D S101E S103A V104I S156D G160S L262E | -4 | 147% | 96% | 110% |
| (b) S9E N43R N76D V205I Q206L Y209W S259D N261W L262E | -3 | 111% | 105% | 107% |
| (c) S9E N43R N76D N185E S188E Q191N A194P Q206L Y209W S259D L262E | -5 | 121% | 93% | 103% |

Table 12: Relative performance of charge mixtures with variant G97D N117R Y209W A215K against variant G97D N117R Y209W A215K alone

| | | Relative performance against variant G97D N 117R Y209W A215K | | |
|---|---|---|---|---|
| Variant | Δ Net charge (rel. to SEQ ID NO: 1) | PC-10 | PC-03 | PC-03 + PC-10 |
| S99D S101E S103A V104I S156D G160S L262E | -4 | 120% | 131% | 126% |
| S9E N43R N76D V205I Q206L Y209W S259D N261W L262E | -3 | 133% | 126% | 129% |
| S9E N43R N76D N185E S188E Q191N A194P Q206L Y209W S259D L262E | -5 | 121% | 118% | 119% |

The data presented in Examples 1 to 5 above demonstrates that the combination of a first protease with a net charge of -5, -4, or-3 compared to SEQ ID NO: 1 and a second protease with a net charge of -1, 0 or +1 compared to SEQ ID NO: 1 results in an improved wash performance. In most cases, the mixtures provide a substantial improvement in wash performance over the single enzymes on both the PC-10 and the PC-03 stains and thus a substantial improvement in the overall wash performance. In a few cases, the mixture has resulted in a poorer performance on PC-03, but this has been outweighed by improved wash performance on PC-10, so that the overall performance of the mixtures on PC-03 + PC-10 is still better than that of the single enzymes.

**Example 6**

Wash performance of SEQ ID NO: 1 or variants thereof with the mutations indicated in Tables 13 and 14 below having a net charge of 0 or -1 relative to SEQ ID NO: 1 were tested in AMSA as described above either alone or in combination with a variant having the mutations S9R S99D S101E S103A V104I S156D G160S Q245R L262E compared to SEQ ID NO: 1 (net charge of -2 relative to SEQ ID NO: 1). The wash performance of the S9R S99D S101E S103A V104I S156D G160S Q245R L262E variant alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 13 the mixtures are compared to single enzymes with a net formal charge of -1 or 0 relative to SEQ ID NO: 1 (enzymes (a), (b), (c) in the column "Variant / single enzyme"), while in Table 14 the mixtures are compared to the variant S9R S99D S101E S103A V104I S156D G160S Q245R L262E alone.

Table 13: Relative performance of charge mixtures with S9R S99D S101E S103A V104I S156D G160S Q245R L262E against single enzymes (a), (b), (c)

| | | Relative performance against single enzyme | | |
|---|---|---|---|---|
| Variant / single enzyme | Δ Net charge (rel. to SEQ ID NO: 1) | PC-10 | PC-03 | PC-03 + PC-10 |
| (a) K27M | -1 | 128% | 93% | 105% |
| (b) SEQ ID NO: 1 | 0 | 126% | 91% | 102% |
| (b) S99AD | -1 | 100% | 112% | 106% |

Table 14: Relative performance of charge mixtures against S9R S99D S101E S103A V104I S156D G160S Q245R L262E alone

| Relative performance against variant S9R S99D S101E S103A | | | | |
|---|---|---|---|---|
| | | V104I S156D G160S Q245R L262E | | |
| Variant (or SEQ ID NO: 1) | Δ Net charge (rel. to SEQ ID NO: 1) | PC-10 | PC-03 | PC-03 + PC-10 |
| K27M | -1 | 132% | 112% | 120% |
| SEQ ID NO: 1 | 0 | 118% | 103% | 109% |
| S99AD | -1 | 169% | 115% | 135% |

**Example 7**

Wash performance of SEQ ID NO: 1 or variants thereof with the mutations indicated in Tables 15 and 16 below were tested in AMSA as described above either alone ("single enzyme") or together with a variant of SEQ ID NO: 1 having the substitutions N76D G97D N140D S156D Y209W A215K L262E (net charge of -4 relative to SEQ ID NO: 1). The wash performance of the N76D G97D N140D S156D Y209W A215K L262E variant alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 15 the mixtures are compared to the single enzymes (enzymes (a), (b), (c) listed in the column "Variant / single enzyme"), while Table 16 compares the mixtures to the N76D G97D N140D S156D Y209W A215K L262E variant

alone.

Table 15: Relative performance of charge mixtures with N76D G97D N140D S156D Y209W A215K L262E against single enzymes (a), (b), (c)

| | | Relative performance against single enzyme | | |
|---|---|---|---|---|
| Variant/single enzyme | Δ Net charge (rel. to SEQ ID NO: 1) | PC-10 | PC-03 | PC-03 + PC-10 |
| (a) SEQ ID NO: 1 | 0 | 126% | 214% | 152% |
| (b) G97D S156D Y209W A215K | -1 | 89% | 127% | 100% |
| (c) G97D Y209WA215K L262E | -1 | 99% | 125% | 108% |

Table 16: Relative performance of charge mixtures against variant N76D G97D N140D S156D Y209WA215K L262E alone

| | | Relative performance against variant N76D G97D N140D S156D Y209W A215K L262E | | |
|---|---|---|---|---|
| Variant (or SEQ ID NO: 1) | Δ Net charge (rel. to SEQ ID NO: 1) | PC-10 | PC-03 | PC-03 + PC-10 |
| SEQ ID NO: 1 | 0 | 125% | 111% | 119% |
| G97D S156D Y209W A215K | -1 | 148% | 110% | 131% |
| G97D Y209W A215K L262E | -1 | 148% | 130% | 140% |

**Example 8**

Wash performance of SEQ ID NO: 1 or variants thereof with the mutations indicated in Tables 17 and 18 below were tested in AMSA as described above either alone ("single enzyme") or together with a variant of SEQ ID NO: 1 having the substitutions K27M N77D G97D S156D Y209W A215K L262E (net charge of -4 relative to SEQ ID NO: 1). The wash performance of the K27M N77D G97D S156D Y209W A215K L262E variant alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 17 the mixtures are compared to the single enzymes (enzymes (a), (b), (c) listed in the column "Variant / single enzyme"), while Table 18 compares the mixtures to the K27M N77D G97D S156D Y209W A215K L262E variant alone.

Table 17: Relative performance of charge mixtures with K27M N77D G97D S156D Y209W A215K L262E against single enzymes (a), (b), (c)

| | | Relative performance against single enzyme | | |
|---|---|---|---|---|
| Variant/single enzyme | Δ Net charge (rel. to SEQ ID NO: 1) | PC-10 | PC-03 | PC-03 + PC-10 |
| (a) SEQ ID NO: 1 | 0 | 132% | 172% | 144% |
| (b) G97D S156D Y209W A215K | -1 | 89% | 116% | 97% |
| (c) G97D Y209WA215K L262E | -1 | 101% | 120% | 107% |

Table 18: Relative performance of charge mixtures against variant K27M N77D G97D S156D Y209WA215K L262E alone

| | | Relative performance against variant K27M N77D G97D S156D Y209W A215K L262E | | |
|---|---|---|---|---|
| Variant (or SEQ ID NO: 1) | Δ Net charge (rel. to SEQ ID NO: 1) | PC-10 | PC-03 | PC-03 + PC-10 |
| SEQ ID NO: 1 | 0 | 123% | 97% | 112% |
| G97D S156D Y209W A215K | -1 | 138% | 109% | 126% |
| G97D Y209W A215K L262E | -1 | 140% | 134% | 138% |

**Example 9**

Wash performance of variants of SEQ ID NO: 1 with the mutations indicated in Tables 19 and 20 below were tested in AMSA as described above either alone ("single enzyme") or together with a variant of SEQ ID NO: 1 having the substitutions G97D S156D Y209W A215K L262E (net charge of -2 relative to SEQ ID NO: 1). The wash performance of the G97D S156D Y209WA215K L262E variant alone was also tested in AMSA.

Determination of delta intensity values for each treatment for each of the two stains PC-10 and PC-03, calculation of the percent relative performance of the mixtures compared to single enzymes, and calculation of the overall performance of the mixtures compared to the single enzymes on the set of stains (PC-03 + PC-10) was performed as described in Example 1.

In Table 19 the mixtures are compared to the single enzymes with a net formal charge of +1, -1, -3 or -4 (enzymes (a), (b), (c), (d), (e), (f), (g), (h), (i) listed in the column "Variant / single enzyme"), while Table 20 compares the mixtures to the variant G97D S156D Y209W A215K L262E alone.

Table 19: Relative performance of charge mixtures with G97D S156D Y209W A215K L262E against single enzymes (a), (b), (c), (d), (e), (f), (g), (h), (i)

| | | Relative performance against single enzyme | | |
|---|---|---|---|---|
| Variant/single enzyme | Δ Net charge (rel. to SEQ ID NO: 1) | PC-10 | PC-03 | PC-03 + PC-10 |
| (a) G97D N117RY209WA215K | 1 | 202% | 382% | 258% |
| (b) G97D S156D Y209W A215K | -1 | 106% | 128% | 115% |
| (c) G97D Y209WA215K L262E | -1 | 119% | 121% | 120% |
| (d) N62D N76D G97D Y209W A215K L262E | -3 | 142% | 125% | 134% |
| (e) N62D G97D S101E V177I Y209W A215K L262E | -3 | 116% | 115% | 115% |
| (f) N62D G97D S101E Y209WA215K L262E | -3 | 120% | 127% | 123% |
| (g) G97D S101E S156D A172V Y209W A215K L262E | -3 | 134% | 117% | 126% |
| (h) K27M N77D G97D S156D Y209W A215K L262E | -4 | 180% | 182% | 181% |
| (i) N76D G97D N140D S156D Y209W A215K L262E | -4 | 180% | 142% | 160% |

Table 20: Relative performance of charge mixtures against variant G97D S156D Y209W A215K L262E alone

| | | Relative performance against variant G97D S156D Y209W A215K L262E | | |
|---|---|---|---|---|
| Variant | Δ Net charge (rel. to SEQ ID NO: 1) | PC-10 | PC-03 | PC-03 + PC-10 |
| G97D N117R Y209WA215K | 1 | 104% | 111% | 107% |
| G97D S156D Y209W A215K | -1 | 118% | 118% | 118% |
| G97D Y209W A215K L262E | -1 | 115% | 118% | 116% |

(continued)

| | | Relative performance against variant G97D S156D Y209W A215K L262E | | |
|---|---|---|---|---|
| Variant | Δ Net charge (rel. to SEQ ID NO: 1) | PC-10 | PC-03 | PC-03 + PC-10 |
| N62D N76D G97D Y209W A215K L262E | -3 | 116% | 114% | 115% |
| N62D G97D S101E V177I Y209W A215K L262E | -3 | 114% | 124% | 119% |
| N62D G97D S101E Y209W A215K L262E | -3 | 103% | 134% | 117% |
| G97D S101E S156D A172V Y209W A215K L262E | -3 | 117% | 123% | 120% |
| K27M N77D G97D S156D Y209W A215K L262E | -4 | 111% | 126% | 118% |
| N76D G97D N140D S156D Y209W A215K L262E | -4 | 111% | 121% | 115% |

**Example 10**

The wash performance of variants of SEQ ID NO: 1 comprising the substitutions G97D+Y209W+A215K and at least one additional substitution as indicated in Table 21 below was tested in the AMSA assay as described above and compared to the performance of the protease of SEQ ID NO: 1.

Determination of delta intensity values for each enzyme for each of the two stains PC-10 and PC-03, calculation of the percent relative performance of the enzymes compared to SEQ ID NO: 1, and calculation of the overall performance of each enzyme compared to SEQ ID NO: 1 on the set of stains (PC-03 + PC-10) was performed as described in Example 1. In this example only individual enzymes were tested. Table 21 shows that all of the tested variants comprising the substitutions G97D+Y209W+A215K exhibited improved wash performance over the reference protease with SEQ ID NO: 1.

Table 21: Relative performance of variants of SEQ ID NO: 1 compared to SEQ ID NO: 1

| | Relative performance against SEQ ID NO: 1 | | |
|---|---|---|---|
| Variant | PC-10 | PC-03 | PC-03 + PC-10 |
| G97D S156D Y209W A215K | 205% | 131% | 169% |
| G97D Y209W A215K L262E | 183% | 142% | 164% |
| G97D S156D Y209W A215K L262E | 160% | 147% | 154% |
| N76D G97D Y209W A215K L262E | 163% | 151% | 157% |
| K27M G97D Y209W A215K L262E | 150% | 142% | 146% |
| N62D G97D Y209W A215K L262E | 155% | 127% | 142% |
| N62D G97D S101E V177I Y209W A215K L262E | 148% | 174% | 161% |
| N62D N76D G97D Y209W A215K L262E | 138% | 137% | 137% |
| K27M N77D G97D S156D Y209W A215K L262E | 108% | 121% | 114% |
| N76D G97D N140D S156D Y209W A215K L262E | 104% | 121% | 112% |

**Example 11**

The wash performance of variants of SEQ ID NO: 1 comprising the substitutions G97D+Y209W+A215K and additional substitutions as indicated in Table 22 below was tested in the AMSA assay as described above and compared to the performance of a variant of SEQ ID NO: 1 with the substitutions S99D+S101E+S103A+V104I+G160S as a reference. This example tests variants of SEQ ID NO: 1 with the substitutions G97D+S156D+Y209W+A215K+L262E and at least one additional substitution selected from S87N, S101G/N, V104N, G118V and A194P.

Determination of delta intensity values for each enzyme for each of the two stains PC-03 and PC-10 and calculation of the percent relative performance of the enzymes compared to the reference protease was performed as described in Example 1. In this example only individual enzymes were tested.

Table 22: Relative performance of variants of SEQ ID NO: 1 compared to SEQ ID NO: 1 + S99D S101E S103A V104I G160S

| | Relative performance compared to SEQ ID NO: 1 + S99D S101E S103A V104I G160S | |
|---|---|---|
| Variant | PC-03 | PC-10 |
| G97D S156D Y209W A215K L262E | 96% | 129% |
| S87N G97D S156D Y209W A215K L262E | 107% | 138% |
| G97D S101G S156D Y209W A215K L262E | 112% | 111% |
| G97D S101N S156D Y209W A215K L262E | 106% | 128% |
| G97D V104N S156D Y209W A215K L262E | 124% | 142% |
| G97D G118V S156D Y209W A215K L262E | 99% | 129% |
| G97D S156D A194P Y209W A215K L262E | 113% | 120% |
| S87N G97D S101G S156D Y209W A215K L262E | 116% | 146% |
| S87N G97D S101N S156D Y209W A215K L262E | 117% | 123% |
| S87N G97D V104N S156D Y209W A215K L262E | 132% | 150% |
| S87N G97D G118V S156D Y209W A215K L262E | 110% | 140% |
| S87N G97D S156D A194P Y209W A215K L262E | 103% | 128% |
| G97D S101G G118V S156D Y209W A215K L262E | 109% | 144% |
| G97D S101G S156D A194P Y209W A215K L262E | 124% | 148% |
| G97D S101N V104N S156D Y209W A215K L262E | 147% | 136% |

Table 22 shows that all of the tested variants exhibited improved wash performance over the reference protease SEQ ID NO: 1 + S99D+S101E+S103A+V104I+G160S, which in itself exhibits good cleaning performance. On the PC-03 stain the tested variants had at least on-par performance, and in most cases improved performance over the reference protease, while on the PC-10 stain all of the tested variants had improved performance over the reference protease. As explained above, the PC-10 stain mimics natural sebum stains, thus providing a good indication of how an enzymatic detergent composition will perform on this difficult to remove natural stain.

**PREFERRED EMBODIMENTS**

1. A detergent composition comprising a first protease and a second protease, wherein:

   1) the first protease has a net formal charge of -1, -3 or -4 relative to the protease of SEQ ID NO: 1, and the second protease has a net formal charge of -2 relative to the protease of SEQ ID NO: 1; or
   2) the first protease has a net formal charge of -3, -4 or -5 relative to the protease of SEQ ID NO: 1, and the second protease has a net formal charge of -1, 0 or +1 relative to the protease of SEQ ID NO: 1.

2. The detergent composition of embodiment 1, wherein the first protease has a net formal charge of -1, -3 or -4 relative to the protease of SEQ ID NO: 1, and the second protease has a net formal charge of -2 relative to the protease of SEQ ID NO: 1, e.g. wherein the first protease has a net formal charge of -3 or -4 relative to the protease of SEQ ID NO: 1.

3. The detergent composition of embodiment 1, wherein the first protease has a net formal charge of -3, -4 or -5 relative to the protease of SEQ ID NO: 1, and the second protease has a net formal charge of -1, 0 or +1 relative to the protease of SEQ ID NO: 1, e.g. wherein the first protease has a net formal charge of -3 relative to the protease of SEQ ID NO: 1, and the second protease has a net formal charge of -1 relative to the protease of SEQ ID NO: 1.

4. The detergent composition of any of the preceding embodiments, comprising a first and a second protease that each have a net formal charge of -1, -2, -3 or -4 relative to the protease of SEQ ID NO: 1, and where the net formal

charge of the first and second protease differ from each other by 1 or 2.

5. The detergent composition of embodiment 4, wherein the composition comprises:

a) a first protease with a net formal charge of -4 and a second protease with a net formal charge of -2 relative to the protease of SEQ ID NO: 1;
b) a first protease with a net formal charge of -3 and a second protease with a net formal charge of -2 relative to the protease of SEQ ID NO: 1; or
c) a first protease with a net formal charge of -3 and a second protease with a net formal charge of -1 relative to the protease of SEQ ID NO: 1.

6. The detergent composition of any of embodiments 1-2 or 4-5, comprising a first protease with a net formal charge of -1, -3 or -4 relative to the protease of SEQ ID NO: 1 and a second protease with a net formal charge of -2 relative to the protease of SEQ ID NO: 1, wherein the second protease is a variant of SEQ ID NO: 1 comprising the substitutions G97D+S156D+Y209W+A215K+L262E or S9R+S99D+S101E+S103A+V104I+S156D+G160S+Q245R+L262E, wherein position numbers are based on the numbering of SEQ ID NO: 2.

7. The detergent composition of any of embodiments 1-2 or 4-6, comprising a first protease with a net formal charge of -1, -3 or -4 relative to the protease of SEQ ID NO: 1 and a second protease with a net formal charge of -2 relative to the protease of SEQ ID NO: 1, wherein the first protease is a variant of SEQ ID NO: 1 having a set of mutations selected from the group consisting of:

- K27M;
- S99AD;
- G97D+S156D+Y209W+A215K;
- G97D+Y209W+A215K+L262E;
- S9R+S99D+S101E+S103A+V104I+S156D+G160S+K235M+Q245R+L262E;
- S9R+S99D+S101E+S103A+V104I+S156D+G160S+L262E;
- S9E+N43R+N76D+V205I+Q206L+Y209W+S259D+N261W+L262E;
- N62D+N76D+G97D+Y209W+A215K+L262E;
- N62D+G97D+S101E+V177I+Y209W+A215K+L262E;
- N62D+G97D+S101E+Y209W+A215K+L262E;
- G97D+S101E+S156D+A172V+Y209W+A215K+L262E;
- S99D+S101E+S103A+V104I+S156D+G160S+L262E;
- K27H+S99D+S101E+S103A+V104I+S156D+G160S+Q245R+L262E;
- S9R+K27M+S99D+S101E+S103A+V104I+S156D+G160S+K237M+Q245R+L262E;
- N76D+G97D+N140D+S156D+Y209W+A215K+L262E; and
- K27M+N77D+G97D+S156D+Y209W+A215K+L262E;

wherein position numbers are based on the numbering of SEQ ID NO: 2

8. The detergent composition of any of embodiments 1 or 3-5 comprising a first protease with a net formal charge of -3, -4 or -5 relative to the protease of SEQ ID NO: 1 and a second protease with a net formal charge of -1, 0 or +1 relative to the protease of SEQ ID NO: 1, wherein the first protease is a variant of SEQ ID NO: 1 comprising a set of mutations selected from the group consisting of:

- S99D+S101E+S103A+V104I+S156D+G160S+L262E;
- K27H+S99D+S101E+S103A+V104I+S156D+G160S+Q245R+L262E;
- S9R+S99D+S101E+S103A+V104I+S156D+G160S+K235M+Q245R+L262E;
- S9R+S99D+S101E+S103A+V104I+S156D+G160S+L262E;
- S9R+K27M+S99D+S101E+S103A+V104I+S156D+G160S+K237M+Q245R+L262E;
- S9E+N43R+N76D+V205I+Q206L+Y209W+S259D+N261W+L262E;
- S9E+N43R+N76D+N185E+S188E+Q191N+A194P+Q206L+Y209W+S259D+L262E;
- *36D+N76D+H120D+G195E+K235L;
- S9R+K27M+S99A+S101E+S103A+V104I+S156D+G160S+K237M+Q245R+L262E;
- S9R+K27M+S101E+S103A+V104I+S156D+G160S+K237M+Q245R+L262E;
- S9R+K27M+S99D+S101E+S103A+V104I+S156D+G160S+A215K+K237M+Q245R+ L262E;
- S9R+K27M+N43R+S99D+S101E+S103A+V104I+S156D+G160S+K237M+Q245R+ L262E;

- S9R+N43R+S99D+S101E+S103A+V104I+S156D+G160S+N173D+K235M+Q245R +L262E;
- S9E+G97D+S156D+Y209W+A215K+L262E;
- G97D+S101E+S156D+A172V+Y209W+A215K+L262E;
- N76D+G97D+N140D+S156D+Y209W+A215K+L262E;
- N76D+G97D+S101E+T180A+Y209W+A215K+L262E;
- N76D+G97D+S101E+Y209W+A215K+L262E;
- N62D+G97D+S101E+Y209W+A215K+L262E;
- N62D+G97D+S101E+V177I+Y209W+A215K+L262E;
- N62D+N76D+G97D+Y209W+A215K+L262E;
- K27M+G97D+S156D+Y209W+A215K+L262E;
- K27M+N77D+G97D+S156D+Y209W+A215K+L262E;
- K27M+N76D+G97D+Y209W+A215K+L262E;
- K27M+N62D+G97D+Y209W+A215K+L262E;
- S9E+G97D+S101E+Y209W+A215K+L262E;
- S9E+N76D+G97D+Y209W+A215K+L262E+A270T;
- S9E+N76D+G97D+Y209W+A215K+L262E;
- S9E+N62D+G97D+Y209W+A215K+L262E;
- S9E+K27M+G97D+Y209W+A215K+L262E;
- S9E+N43R+N76D+N185E+S188E+Q191N+A194P+Q206L+Y209W+A215K+Q245R +S259D+L262E;
- S9E+N43R+N76D+S188E+Q191N+A194P+Q206L+Y209W+A215K+S259D+L262E;
- S9E+N43R+N76D+N185E+S188E+Q191N+A194P+Q206L+Y209W+A215K+S259D +L262Q; and
- S9E+N43R+N76D+N185E+S188E+Q191N+A194P+Q206L+Y209W+Q245R+S259D +L262Q;

wherein position numbers are based on the numbering of SEQ ID NO: 2.

9. The detergent composition of any of embodiments 1, 3-5 or 8 comprising a first protease with a net formal charge of -3, -4 or -5 relative to the protease of SEQ ID NO: 1 and a second protease with a net formal charge of -1, 0 or +1 relative to the protease of SEQ ID NO: 1, wherein the second protease is the protease of SEQ ID NO: 1 or a variant of SEQ ID NO: 1 comprising a set of mutations selected from the group consisting of:

- K27M;
- S99AD;
- G97D+Y209W+A215K;
- G97D+N117R+Y209W+A215K;
- S9R+K27M+N43R+N76D+V205I+Q206L+Y209W+A215K+Q245R+S259D+N261W+ L262E;
- S9R+N43R+N76D+V205I+Q206L+Y209W+A215K+S259D+N261W+L262E;
- S9R+N43R+N76D+V205I+Q206L+Y209W+S259D+N261W+L262E;
- S9R+S99A+S101E+S103A+V104I+S156D+G160S+A215K+K235M+Q245R+L262E;
- S9R+K27M+N43R+S99A+S101E+S103A+V104I+S156D+G160S+A215K+K237M+ Q245R+L262E;
- S9R+K27M+N43R+S101E+S103A+V104I+S156D+G160S+A215K+K237M+Q245R +L262E;
- Y167A+R170S+A194P;
- S9R+A15T+V68A+N218D+Q245R;
- S9R+A15T+G61E+V68A+A194P+V205I+Q245R+N261D;
- S9R+A15T+V68A+S99G+Q245R+N261D;
- S9R+A15T+V68A+H120D+P131S+Q137H+Q245R;
- S9R+A15T+V68A+H120N+P131S+Q137H+Q245M;
- S9R+A15T+G61E+V68A+A98S+S99G+N218D+Q245R;
- S3T+V4I+S99D+S101R+S103A+V104I+G160S+V2051+L217D;
- S3T+V4I+S99D+S101R+S103A+V104I+G160S+A194P+V205I+L217D;
- G97D+Y209W+A215K+L262E; and
- G97D+S156D+Y209W+A215K;

wherein position numbers are based on the numbering of SEQ ID NO: 2.

10. The detergent composition of any of the preceding embodiments, wherein the first protease and the second protease each have an amino acid sequence that has at least 60% sequence identity to SEQ ID NO: 1, for example at least 70%, at least 80%, at least 85% or at least 90% sequence identity to SEQ ID NO: 1.

11. The detergent composition of any of the preceding embodiments, wherein the composition is a laundry detergent composition.

12. The detergent composition of embodiment 11, wherein the composition is a liquid laundry detergent composition.

13. The detergent composition of any of the preceding embodiments, wherein the composition has an improved wash performance in laundry compared to the same composition comprising either the first protease or the second protease; e.g. wherein the composition has an improved wash performance on the PC-10 stain compared to the same composition comprising either the first protease or the second protease, and/or wherein the composition has an improved overall wash performance on a set of standard stains comprising at least PC-10 and PC-03 compared to the same composition comprising either the first protease or the second protease.

14. Use of a composition according to any of embodiments 1-13 in a cleaning process, in particular for laundry.

15. A method of cleaning, especially for cleaning fabrics or textiles, comprising contacting a fabrics or textile with a detergent composition according to any of embodiments 1-13 under conditions suitable for cleaning the fabrics or textile.

16. A protease variant comprising the substitutions X97D, X209W and X215K, e.g. G97D, Y209W and A215K, wherein position numbers are based on the numbering of SEQ ID NO: 2, and the variant has protease activity and has at least 80% but less than 100% sequence identity to SEQ ID NO: 1.

17. The protease variant of embodiment 16, wherein the protease is a variant of SEQ ID NO: 1 comprising a set of substitutions selected from the group consisting of:

G97D+Y209W+A215K;
G97D+S156D+Y209W+A215K;
G97D+Y209W+A215K+L262E;
G97D+N117R+Y209W+A215K;
S9E+G97D+S156D+Y209W+A215K+L262E;
N62D+G97D+Y209W+A215K+L262E;
G97D+S101E+S156D+A172V+Y209W+A215K+L262E;
N76D+G97D+N140D+S156D+Y209W+A215K+L262E;
N76D+G97D+S101E+T180A+Y209W+A215K+L262E;
N76D+G97D+S101E+Y209W+A215K+L262E;
N62D+G97D+S101E+Y209W+A215K+L262E;
N62D+G97D+S101E+V177I+Y209W+A215K+L262E;
N62D+N76D+G97D+Y209W+A215K+L262E;
K27M+G97D+S156D+Y209W+A215K+L262E;
K27M+N77D+G97D+S156D+Y209W+A215K+L262E;
K27M+N76D+G97D+Y209W+A215K+L262E;
K27M+N62D+G97D+Y209W+A215K+L262E;
S9E+G97D+S101E+Y209W+A215K+L262E;
S9E+N76D+G97D+Y209W+A215K+L262E+A270T;
S9E+N76D+G97D+Y209W+A215K+L262E;
S9E+N62D+G97D+Y209W+A215K+L262E;
S9E+K27M+G97D+Y209W+A215K+L262E;
G97D+S156D+Y209W+A215K+L262E;
G97D+S101E+Y209W+A215K+L262E;
N76D+G97D+Y209W+A215K+L262E;
K27M+G97D+Y209W+A215K+L262E;
S9E+G97D+Y209W+A215K+L262E;
S87N+G97D+S156D+Y209W+A215K+L262E;
G97D+S101G+S156D+Y209W+A215K+L262E;
G97D+S101 N+S156D+Y209W+A215K+L262E;
G97D+V104N+S156D+Y209W+A215K+L262E;
G97D+G118V+S156D+Y209W+A215K+L262E;
G97D+S156D+A194P+Y209W+A215K+L262E;

S87N+G97D+S101G+S156D+Y209W+A215K+L262E;
S87N+G97D+S101N+S156D+Y209W+A215K+L262E;
S87N+G97D+V104N+S156D+Y209W+A215K+L262E;
S87N+G97D+G118V+S156D+Y209W+A215K+L262E;
S87N+G97D+S156D+A194P+Y209W+A215K+L262E;
G97D+S101G+G118V+S156D+Y209W+A215K+L262E;
G97D+S101G+S156D+A194P+Y209W+A215K+L262E; and
G97D+S101N+V104N+S156D+Y209W+A215K+L262E;

wherein position numbers are based on the numbering of SEQ ID NO: 2.

## Claims

1. A protease variant comprising the substitutions X97D, X209W, and X215K (e.g. G97D, Y209W, and A215K), wherein position numbers are based on the numbering of SEQ ID NO: 2, and wherein the variant has protease activity and has at least 80% but less than 100% sequence identity to SEQ ID NO: 1.

2. The protease variant according to claim 1, which has at least 85%, such as at least 90%, or at least 95%, but less than 100%, sequence identity to SEQ ID NO: 1.

3. The protease variant according to claim 1 or 2, which further comprises one or more additional substitutions selected from the group consisting of X9E (e.g. S9E), X27M (e.g. K27M), X62D (e.g. N62D), X76D (e.g. N76D), X77D (e.g. N77D), X87N (e.g. S87N), X101E/G/N (e.g. S101E/G/N), X104N (e.g. V104N), X117R (e.g. N117R), X118V (e.g. G118V), X140D (e.g. N140D), X156D (e.g. S156D), X172V (e.g. A172V), X1771 (e.g. V1771), X194P (e.g. A194P), and X262E (e.g. L262E)

4. The protease variant according to any of claims 1-3, wherein the protease variant is a variant of SEQ ID NO: 1 comprising a set of substitutions selected from the group consisting of:

G97D+Y209W+A215K;
G97D+S156D+Y209W+A215K;
G97D+Y209W+A215K+L262E;
G97D+N117R+Y209W+A215K;
S9E+G97D+S156D+Y209W+A215K+L262E;
N62D+G97D+Y209W+A215K+L262E;
G97D+S101E+S156D+A172V+Y209W+A215K+L262E;
N76D+G97D+N140D+S156D+Y209W+A215K+L262E;
N76D+G97D+S101E+T180A+Y209W+A215K+L262E;
N76D+G97D+S101E+Y209W+A215K+L262E;
N62D+G97D+S101E+Y209W+A215K+L262E;
N62D+G97D+S101E+V177I+Y209W+A215K+L262E;
N62D+N76D+G97D+Y209W+A215K+L262E;
K27M+G97D+S156D+Y209W+A215K+L262E;
K27M+N77D+G97D+S156D+Y209W+A215K+L262E;
K27M+N76D+G97D+Y209W+A215K+L262E;
K27M+N62D+G97D+Y209W+A215K+L262E;
S9E+G97D+S101E+Y209W+A215K+L262E;
S9E+N76D+G97D+Y209W+A215K+L262E+A270T;
S9E+N76D+G97D+Y209W+A215K+L262E;
S9E+N62D+G97D+Y209W+A215K+L262E;
S9E+K27M+G97D+Y209W+A215K+L262E;
G97D+S156D+Y209W+A215K+L262E;
G97D+S101E+Y209W+A215K+L262E;
N76D+G97D+Y209W+A215K+L262E;
K27M+G97D+Y209W+A215K+L262E;
S9E+G97D+Y209W+A215K+L262E;
S87N+G97D+S156D+Y209W+A215K+L262E;

G97D+S101G+S156D+Y209W+A215K+L262E;
G97D+S101 N+S156D+Y209W+A215K+L262E;
G97D+V104N+S156D+Y209W+A215K+L262E;
G97D+G118V+S156D+Y209W+A215K+L262E;
G97 D+S 156 D+A 194 P+Y209W+A215K+L262 E;
S87N+G97D+S101G+S156D+Y209W+A215K+L262E;
S87N+G97D+S101N+S156D+Y209W+A215K+L262E;
S87N+G97D+V104N+S156D+Y209W+A215K+L262E;
S87N+G97D+G118V+S156D+Y209W+A215K+L262E;
S87N+G97D+S156D+A194P+Y209W+A215K+L262E;
G97D+S101G+G118V+S156D+Y209W+A215K+L262E;
G97D+S101G+S156D+A194P+Y209W+A215K+L262E; and
G97D+S101N+V104N+S156D+Y209W+A215K+L262E;

wherein position numbers are based on the numbering of SEQ ID NO: 2.

**5.** A detergent composition comprising a protease variant according to any of claims 1-4.

**6.** The detergent composition according to claim 5 in the form of a homogenous tablet, a tablet having two or more layers, a pouch having one or more compartments, a regular or compact powder, a granule, a paste, a gel, a bar, or a regular, compact, or concentrated liquid.

**7.** The detergent composition according to claim 5 or 6, which further comprises an amylase, an arabinase, a carbohydrase, a cellulase (e.g. endoglucanase), a cutinase, a deoxyribonuclease, a galactanase, a haloperoxygenase, a lipase, a mannanase, an oxidase (e.g., a laccase and/or peroxidase), a pectinase, a pectin lyase, an additional protease, a xylanase, a xanthanase, a xyloglucanase, or any mixture thereof.

**8.** The detergent composition according to claim 7, wherein the additional protease has a different net formal charge.

**9.** Use of a protease variant according to any of claims 1-4 in a cleaning process, preferably laundry.

```
SEQ01        1 AQSVPWGISRVQAPAAHNRGLTGSGVKVAVLDTGI-STHPDLNIRGGASF  49
               ||||| | |   ||| |  | ||| ||||| | || | ||||   ||||
SEQ02        1 AQSVPYGVSQIKAPALHSQGYTGSNVKVAVIDSGIDSSHPDLKVAGGASM  50

SEQ01       50 VPGEP-STQDGNGHGTHVAGTIAALNNSIGVLGVAPSAELYAVKVLGASG  98
               || |    || | |||||||| |||||||||||||||| ||||||||| |
SEQ02       51 VPSETNPFQDNNSHGTHVAGTVAALNNSIGVLGVAPSASLYAVKVLGADG 100

SEQ01       99 SGSVSSIAQGLEWAGNNGMHVANLSLGSPSPSATLEQAVNSATSRGVLVV 148
               ||  | |  | |||  | | | | ||| || || |  ||  |   || ||
SEQ02      101 SGQYSWIINGIEWAIANNMDVINMSLGGPSGSAALKAAVDKAVASGVVVV 150

SEQ01      149 AASGNSGA-GSIS---YPARYANAMAVGATDQNNNRASFSQYGAGLDIVA 194
               || || |  || |   ||  |    |||| |  | |||||  |  ||  |
SEQ02      151 AAAGNEGTSGSSSTVGYPGKYPSVIAVGAVDSSNQRASFSSVGPELDVMA 200

SEQ01      195 PGVNVQSTYPGSTYASLNGTSMATPHVAGAAALVKQKNPSWSNVQIRNHL 244
               |||  ||| ||  |   |||||| |||||||||   | | | | | |  |
SEQ02      201 PGVSIQSTLPGNKYGAYNGTSMASPHVAGAAALILSKHPNWTNTQVRSSL 250

SEQ01      245 KNTATSLGSTNLYGSGLVNAEAATR    269
                || | ||    || || |  ||
SEQ02      251 ENTTTKLGDSFYYGKGLINVQAAAQ    275
```

## FIGURE 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009021867 A
- WO 2014177430 A
- WO 2016000970 A
- WO 2016000971 A
- WO 2016087619 A
- WO 8906279 A
- US 20170198243 A
- WO 9219709 A
- WO 9219708 A
- WO 2005105826 A
- WO 2009118375 A
- WO 2009102854 A
- US 5977053 A
- WO 9817767 A
- EP 624154 A
- WO 2007087258 A
- WO 2007087244 A
- WO 2007087259 A
- WO 2007087242 A
- WO 2006130575 A
- WO 2005003274 A
- WO 2005003275 A
- WO 2005003276 A
- EP 1876226 A
- WO 2007087257 A
- WO 2007087243 A
- US 4435307 A
- WO 9629397 A
- US 5648263 A
- US 5691178 A
- US 5776757 A
- WO 8909259 A
- WO 9117244 A
- WO 02099091 A
- JP 2000210081 B
- EP 0495257 A
- EP 0531372 A
- WO 9611262 A
- WO 9808940 A
- WO 9407998 A
- EP 0531315 A
- US 5457046 A
- US 5686593 A
- US 5763254 A
- WO 9524471 A
- WO 9812307 A
- WO 2002099091 A
- WO 2001062903 A
- WO 1999064619 A
- WO 2007044993 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 2010065455 A
- WO 2010107560 A
- US 5389536 A
- WO 2011084412 A
- WO 2011084417 A
- WO 2011084599 A
- WO 2011150157 A
- WO 2012137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 9707202 A
- WO 0034450 A
- WO 0060063 A
- WO 0192502 A
- WO 200787508 A
- WO 2009109500 A
- WO 2010111143 A
- WO 2005056782 A
- WO 2009067279 A
- WO 2010100028 A
- GB 1296839 A
- WO 9510603 A
- WO 9402597 A
- WO 9418314 A
- WO 9743424 A
- WO 9919467 A
- WO 0210355 A
- WO 2006066594 A
- WO 9623873 A
- WO 2008153815 A
- WO 0166712 A

- WO 2009061380 A
- WO 2013184577 A
- WO 2010104675 A
- WO 2011098531 A
- WO 2013001078 A
- WO 2013001087 A
- WO 2016180748 A
- WO 2018141707 A
- WO 2017191160 A
- WO 9324618 A
- WO 9510602 A
- WO 9815257 A
- WO 2011098579 A
- WO 2014087011 A
- WO 2009087523 A
- WO 2007138054 A
- WO 2006108856 A
- WO 2006113314 A
- EP 1867808 A
- WO 03040279 A
- US 20090011970 A
- WO 2013007594 A
- WO 2009092699 A
- EP 1705241 A
- EP 1382668 A
- WO 2007001262 A
- US 6472364 B
- WO 2004074419 A
- WO 2013188331 A
- WO 9813459 A
- WO 2017207762 A
- WO 0242740 A

**Non-patent literature cited in the description**

- Enzyme Nomenclature. NC-IUBMB. Academic Press, 1992
- *Eur. J. Biochem.*, 1994, vol. 223, 1-5
- *Eur. J. Biochem.*, 1995, vol. 232, 1-6
- *Eur. J. Biochem.*, 1996, vol. 237, 1-5
- *Eur. J. Biochem.*, 1997, vol. 250, 1-6
- *Eur. J. Biochem.*, 1999, vol. 264, 610-650
- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **EDGAR**. *Nucleic Acids Research*, 2004, vol. 32, 1792-1797
- **KATOH** ; **KUMA**. *Nucleic Acids Research*, 2002, vol. 30, 3059-3066
- **KATOH et al.** *Nucleic Acids Research*, 2005, vol. 33, 511-518
- **KATOH** ; **TOH**. *Bioinformatics*, 2007, vol. 23, 372-374
- **KATOH et al.** *Methods in Molecular Biology*, 2009, vol. 537, 39-64
- **KATOH** ; **TOH**. *Bioinformatics*, 2010, vol. 26, 1899-1900
- **THOMPSON et al.** *Nucleic Acids Research*, 1994, vol. 22, 4673-4680
- **H. NEURATH** ; **R.L. HILL**. The Proteins. Academic Press, 1979
- **CUNNINGHAM** ; **WELLS**. *Science*, 1989, vol. 244, 1081-1085
- **HILTON et al.** *J. Biol. Chem.*, 1996, vol. 271, 4699-4708
- **DE VOS et al.** *Science*, 1992, vol. 255, 306-312
- **SMITH et al.** *J. Mol. Biol.*, 1992, vol. 224, 899-904
- **WLODAVER et al.** *FEBS Lett.*, 1992, vol. 309, 59-64
- **HODGDON** ; **KALER**. *Current Opinion in Colloid & Interface Science*, 2007, vol. 12, 121-128
- Powdered Detergents. Surfactant Science Series. Marcel Dekker, Inc., 1997, vol. 71
- Powdered Detergents. Surfactant science series. Marcel Dekker, Inc., vol. 71
- **SAMBROOK et al.** Molecular Cloning. Cold Spring Harbor Laboratory Press, 2001